# EUROPEAN PATENT APPLICATION

(11) **EP 4 190 768 A1**
(43) Date of publication of application: **07.06.2023**
(21) Application number: 21852994.9
(22) Date of filing: 02.08.2021
(51) Int. Cl.: C07C 215/68, C07C 213/00, C08G 73/10, C08G 73/22, H01B 3/30

(54) **FLUORINATED DIAMINE OR SALT THEREOF, METHOD FOR PRODUCING FLUORINATED DIAMINE OR SALT THEREOF, POLYAMIDE, METHOD FOR PRODUCING POLYAMIDE, POLYAMIDE SOLUTION, CYCLIZED POLYAMIDE, METHOD FOR PRODUCING CYCLIZED POLYAMIDE, INSULATING MATERIAL FOR HIGH-FREQUENCY ELECTRONIC COMPONENT, METHOD FOR PRODUCING INSULATING MATERIAL FOR HIGH-FREQUENCY ELECTRONIC COMPONENT, HIGH-FREQUENCY ELECTRONIC COMPONENT, HIGH-FREQUENCY APPLIANCE, AND INSULATING MATERIAL FOR PRODUCING HIGH-FREQUENCY ELECTRONIC COMPONENT**

(30) Priority: 05.08.2020 JP 2020132901
(71) Applicant: Central Glass Company, Limited, Ube-shi, Yamaguchi 755-0001 (JP)
(72) Inventor: EGUCHI, Hiroshi, Tokyo 101-0054 (JP); HAGIWARA, Yuki, Tokyo 101-0054 (JP); YAMADA, Masaki, Tokyo 101-0054 (JP); YAMANAKA, Kazuhiro, Tokyo 101-0054 (JP); MURAKAMI, Yosuke, Tokyo 101-0054 (JP); HOSOI, Kenji, Tokyo 101-0054 (JP)
(74) Representative: Manitz Finsterwald Patent- und Rechtsanwaltspartnerschaft mbB
(86) International application number: PCT/JP2021/028614
(87) International publication number: WO 2022/030447

(57) **Abstract**

Provided are a fluorinated diamine represented by General Formula [1A] or a salt of the fluorinated diamine; a polyamide including a structural unit derived from the diamine or the salt thereof; a cyclized polyamide; and the like. In General Formula [1A], in a case where a plurality of R¹'s are present, the plurality of R¹'s are each independently at least one selected from the group consisting of an alkyl group, an alkoxy group, a halogen atom, a haloalkyl group, and a haloalkoxy group, and two n's are each independently an integer of 0 to 3.

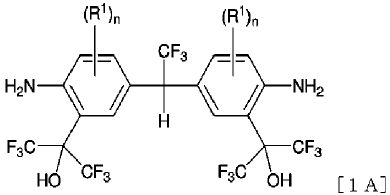

## Description

### TECHNICAL FIELD

The present invention relates to a fluorinated diamine or a salt thereof, a method for producing a fluorinated diamine or a salt thereof, a polyamide, a method for producing a polyamide, a polyamide solution, a cyclized polyamide, a method for producing a cyclized polyamide, an insulation for a high-frequency electronic component, a method for producing an insulation for a high-frequency electronic component, a high-frequency electronic component, a high-frequency appliance, and an insulating material for producing a high-frequency electronic component.

### BACKGROUND ART

A fluorine-containing polymer may exhibit better dielectric properties than a non-fluorine-containing polymer due to electronic peculiarities of the fluorine atom. Therefore, there are cases where attempts are made to use the fluorine-containing polymer, for example, as a material for producing a high-frequency electronic component.

For example, Patent Document 1 discloses a fluorine-containing polyimide film having a dielectric loss tangent of 0.007 or less, a water absorption rate of 0.8% or less, and a linear expansion coefficient at 50°C to 200°C of 30 ppm/°C or less. According to the disclosure of Patent Document 1, the fluorine-containing polyimide film has properties such as low dielectric constant and low water absorption (and low permeability to water vapor and gas), and is particularly suitable for high-frequency substrates.

### RELATED DOCUMENT

### PATENT DOCUMENT

[Patent Document 1] Japanese Unexamined Patent Publication No. 2018-165346

### SUMMARY OF THE INVENTION

### TECHNICAL PROBLEM

As the fluorine-containing polymer, various polymers such as the polyamide disclosed in Patent Document 1 have been known. However, there is a potential demand for a novel fluorine-containing polymer from the viewpoint of increased degree of freedom in material selection and good performance (for example, good heat resistance and dielectric properties).

The present invention has been made in view of such circumstances . An object of the present invention is to provide a novel fluorine-containing polymer which can be preferably applied, for example, to a production of a high-frequency electronic component.

### SOLUTION TO PROBLEM

The present inventors have completed the invention provided below, thereby solving the above-described problems.

According to the present invention, a fluorinated diamine represented by General Formula [1A] or a salt of the fluorinated diamine is provided.

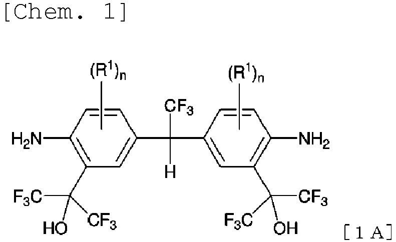

In General Formula [1A],
in a case where a plurality of R¹'s are present, the plurality of R¹'s are each independently at least one selected from the group consisting of an alkyl group, an alkoxy group, a halogen atom, a haloalkyl group, and a haloalkoxy group, and
two n's are each independently an integer of 0 to 3.

In addition, according to the present invention, there is provided a method for producing the above-described fluorinated diamine or the above-described salt of the fluorinated diamine, the method including: a step of adding hexafluoroacetone or an equivalent of hexafluoroacetone to an aromatic diamine compound represented by General Formula [2].

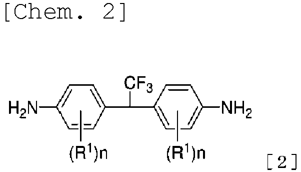

In General Formula [2], definitions of R¹ and n are the same as definitions of R¹ and n in General Formula [1A].

In addition, according to the present invention, a polyamide having a structural unit represented by General Formula [1B] is provided.

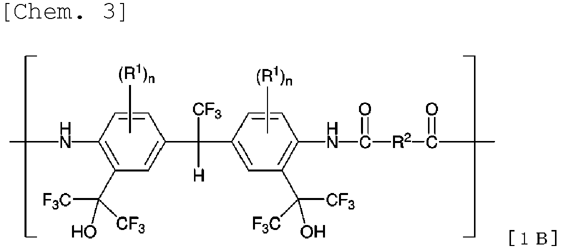

In General Formula [1B],
in a case where a plurality of R¹'s are present, the plurality of R¹'s are each independently at least one selected from the group consisting of an alkyl group, an alkoxy group, a halogen atom, a haloalkyl group, and a haloalkoxy group,
R² is a divalent organic group, and
two n's are each independently an integer of 0 to 3.

In addition, according to the present invention, a polyamide solution containing the above-described polyamide and an organic solvent is provided.

In addition, according to the present invention, a cyclized polyamide having a structural unit represented by General Formula [1C] is provided.

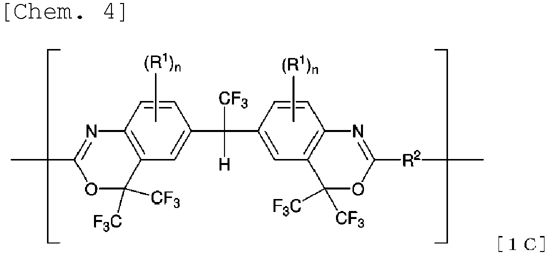

In General Formula [1C],
in a case where a plurality of R¹'s are present, the plurality of R¹'s are each independently at least one selected from the group consisting of an alkyl group, an alkoxy group, a halogen atom, a haloalkyl group, and a haloalkoxy group,
R² is a divalent organic group, and
two n's are each independently an integer of 0 to 3.

In addition, according to the present invention, an insulation for a high-frequency electronic component, including the above-described cyclized polyamide, is provided.

In addition, according to the present invention, a high-frequency electronic component including the above-described insulation for a high-frequency electronic component is provided.

In addition, according to the present invention, a high-frequency appliance including the above-described high-frequency electronic component is provided.

In addition, according to the present invention, an insulating material for producing a high-frequency electronic component, including the above-described polyamide, is provided.

In addition, according to the present invention, a production method for producing the above-described polyamide, the production method including a step of polycondensing a fluorinated diamine represented by General Formula [1A] or a salt of the fluorinated diamine with a dicarboxylic acid represented by General Formula [DC1] or [DC2] or a derivative of the dicarboxylic acid, is provided.

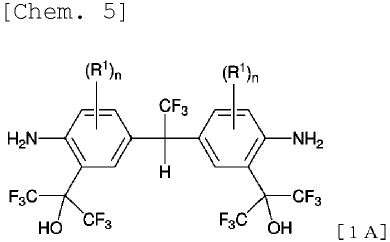

In General Formula [1A],
in a case where a plurality of R¹'s are present, the plurality of R¹'s are each independently at least one selected from the group consisting of an alkyl group, an alkoxy group, a halogen atom, a haloalkyl group, and a haloalkoxy group, and
two n's are each independently an integer of 0 to 3.

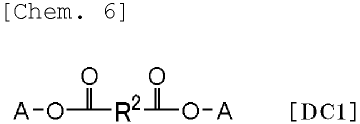

In General Formula [DC1], R² is the same as R² in General Formula [1B], and two A's each independently represent a hydrogen atom, an alkyl group having 1 to 10 carbon atoms, or an aromatic hydrocarbon group having 6 to 10 carbon atoms.

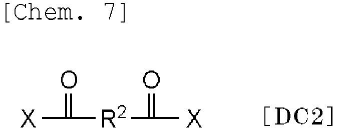

In General Formula [DC2], R² is the same as R² in General Formula [1B], and two X's each independently represent a fluorine atom, a chlorine atom, a bromine atom, an iodine atom, or an active ester group.

In addition, according to the present invention, a production method for producing the above-described cyclized polyamide, the production method including a first step of producing a polyamide by the above-described production method for producing the polyamide and a second step of dehydrating and ring-closing the polyamide obtained in the first step.

In addition, according to the present invention, a method for producing an insulation for a high-frequency electronic component, the method including a coating step of applying the above-described polyamide solution onto a supporting base material, a drying step of drying the solvent contained in the applied polyamide solution to obtain a resin film including the polyamide, and a heating step of heat-treating the resin film to form a cured film, is provided.

### ADVANTAGEOUS EFFECTS OF INVENTION

According to the present invention, a novel fluorine-containing polymer which can be preferably applied, for example, to a production of a high-frequency electronic component is provided.

### DESCRIPTION OF EMBODIMENTS

Hereinafter, embodiments of the present invention will be described in detail.

In the present specification, the notation "X to Y" in the description of numerical range means X or more and Y or less, unless otherwise specified. For example, "1% to 5% by mass" means "1% by mass or more and 5% by mass or less".

In the notation of groups (atomic groups) in the present specification, a notation without indicating whether it is substituted or unsubstituted includes both those having no substituent and those having a substituent. For example, a term "alkyl group" includes not only alkyl groups having no substituent (unsubstituted alkyl groups) but also alkyl groups having a substituent (substituted alkyl groups).

A term "organic group" used in the present specification means an atomic group obtained by removing one or more hydrogen atoms from an organic compound, unless otherwise specified. For example, a "monovalent organic group" represents an atomic group obtained by removing one hydrogen atom from an optional organic compound.

In the chemical formulae in the present specification, a notation "Me" represents a methyl group (CH₃).

In the present specification, a term "fluoral" means trifluoroacetaldehyde.

A term "high frequency" in the present specification means, for example, a frequency range of 1 GHz or more, preferably 10 to 200 GHz and more preferably 28 to 100 GHz.

### <Fluorinated diamine or salt thereof>

The fluorinated diamine according to the present embodiment is represented by General Formula [1A] below. In addition, the salt of the fluorinated diamine according to the present embodiment is obtained by neutralizing an amino group moiety in the fluorinated diamine represented by General Formula [1A] with an acid, or obtained by neutralizing a -C(CF₃)₂OH moiety with a base. The salt is preferably the former, and examples of such a salt include hydrochlorides, sulfates, and nitrates.

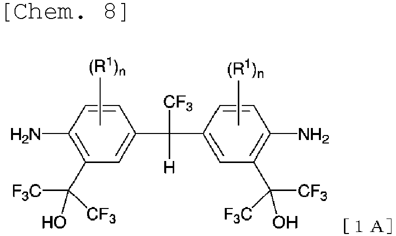

In General Formula [1A],
in a case where a plurality of R¹'s are present, the plurality of R¹'s are each independently at least one selected from the group consisting of an alkyl group, an alkoxy group, a halogen atom, a haloalkyl group, and a haloalkoxy group, and
two n's are each independently an integer of 0 to 3.

By polycondensing the fluorinated diamine or the salt thereof according to the present embodiment with a dicarboxylic acid or a derivative thereof, a fluorine-containing polyamide resin can be produced. In addition, a cyclized polyamide can be derived from the fluorine-containing polyamide resin. The cyclized polyamide tends to have good dielectric properties and heat resistance.

Details of the polyamide resin and the cyclized polyamide will be described later.

The alkyl group of R¹ may be linear or branched. Specific examples of the alkyl group include linear or branched alkyl groups having 1 to 6 carbon atoms. Among these, an n-butyl group, an s-butyl group, an isobutyl group, a t-butyl group, an n-propyl group, an i-propyl group, an ethyl group, or a methyl group is preferable, and an ethyl group or a methyl group is more preferable.

The alkoxy group of R¹ may be linear or branched. Specific examples of the alkoxy group include linear or branched alkoxy groups having 1 to 6 carbon atoms. Among these, an n-butoxy group, an s-butoxy group, an isobutoxy group, a t-butoxy group, an n-propoxy group, an i-propoxy group, an ethoxy group, or a methoxy group is preferable, and an ethoxy group or a methoxy group is particularly preferable.

Examples of the halogen atom of R¹ include a fluorine atom, a chlorine atom, a bromine atom, and an iodine atom, and a fluorine atom is preferable.

Examples of the haloalkyl group and the haloalkoxy group of R¹ include those in which some or all of hydrogen atoms in the above-described alkyl group or alkoxy group are substituted with halogen atoms (preferably, fluorine atoms).

In General Formula [1A], n is preferably an integer of 0 to 2 and more preferably an integer of 0 or 1.

From the cost of raw materials and ease of synthesis, preferred examples of the fluorinated diamine represented by General Formula [1A] or the salt thereof include a fluorinated diamine represented by General Formula [1A-1], [1A-2], or [1A-3] or a salt thereof.

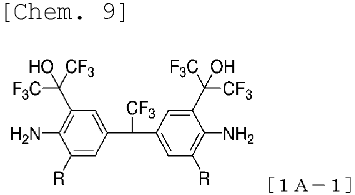

In General Formula [1A-1], two R's are each independently an alkyl group having 1 to 6 carbon atoms.

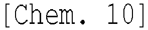

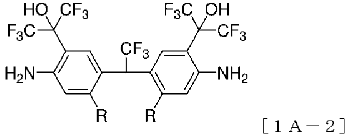

In General Formula [1A-2], two R's are each independently an alkyl group having 1 to 6 carbon atoms.

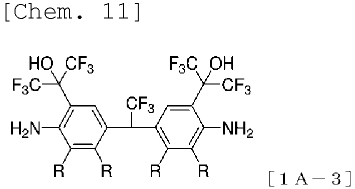

In General Formula [1A-3], four R's are each independently an alkyl group having 1 to 6 carbon atoms.

In particular, among the compounds represented by General Formula [1A], fluorinated diamines represented by the following formulas [1A-4] to [1A-7] or salts thereof can be produced with particularly high purity and high yield by the method described above .

The compound name of the diamine represented by Formula [1A-4] is
1,1,1-trifluoro-2,2-bis(3-(1-hydroxy-1-trifluoromethyl-2,2,2-tri fluoroethyl)-4-aminophenyl) ethane.

The compound name of the diamine represented by Formula [1A-5] is
1,1,1-trifluoro-2,2-bis(3-(1-hydroxy-1-trifluoromethyl-2,2,2-tri fluoroethyl)-5-methyl-4-aminophenyl) ethane.

The compound name of the diamine represented by Formula [1A-6] is
1,1,1-trifluoro-2,2-bis(3-(1-hydroxy-1-trifluoromethyl-2,2,2-tri fluoroethyl)-6-methyl-4-aminophenyl)ethane.

The compound name of the diamine represented by Formula [1A-7] is
1,1,1-trifluoro-2,2-bis(3-(1-hydroxy-1-trifluoromethyl-2,2,2-tri fluoroethyl)-5,6-dimethyl-4-aminophenyl)ethane.

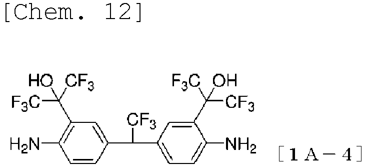

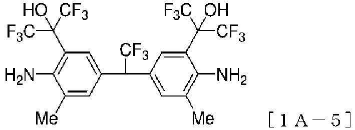

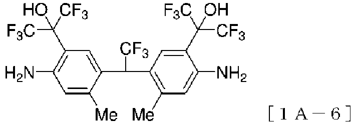

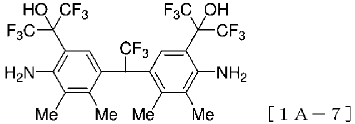

### <Method for producing fluorinated diamine or salt thereof>

The fluorinated diamine represented by General Formula [1A] or the salt thereof can be obtained by, as shown in the following reaction formula, adding hexafluoroacetone (hereinafter, may be represented as "HFA") or an equivalent thereof to an aromatic diamine compound represented by General Formula [2].

In General Formula [2], definitions of R¹ and n are the same as definitions of R¹ and n in General Formula [1A].

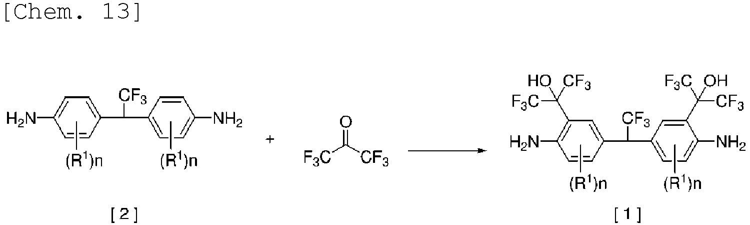

The aromatic diamine compound represented by General Formula [2] can be obtained by, as shown in the following reaction formula, reacting a mixture of fluoral and hydrogen fluoride with an amine compound represented by General Formula [3].

In the following reaction formula, definitions of R¹ and n are the same as definitions of R¹ and n in General Formula [1A].

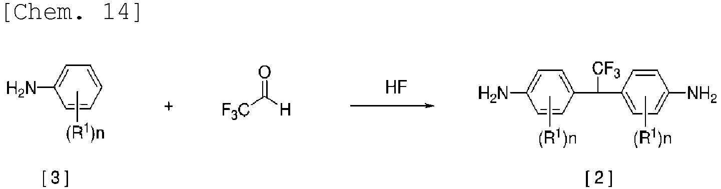

As the fluoral as a starting raw material, a hydrate or fluoral hemiacetal of a commercially available product (product manufactured by Tokyo Chemical Industry Co., Ltd.) can be used as an equivalent thereof. In addition, a hydrate of fluoral or a hemiacetal of fluoral can be prepared by the method described in Japanese Unexamined Patent Publication No. H5-97757 and the like, and can be used.

In general, since the fluoral is usually used as a hydrate or a hemiacetal, in a case where the fluoral is used under anhydrous conditions, anhydrous fluoral can be prepared by dehydrating the hydrate or hemiacetal of fluoral.

On the other hand, as described in Japanese Unexamined Patent Publication No. H3-184933, catalytic gas-phase fluorination of inexpensive chloral can be converted almost quantitatively to fluoral, which can also be used to prepare anhydrous fluoral (see also Preparation Example 1 later for this).

As described in Japanese Unexamined Patent Publication No. 2019-026628, the fluoral is a low-boiling-point compound, generally highly self-reactive and difficult to handle, but the fluoral can be handled very stably in a hydrogen fluoride solution. In a case where the fluoral is treated in hydrogen fluoride, 1,2,2,2-tetrafluoroethanol, which is an adduct of fluoral and hydrogen fluoride, is produced as shown in the scheme below (see also Preparation Example 1 later for this).

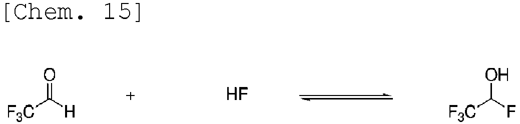

As described above, 1,2,2,2-tetrafluoroethanol forms an equilibrium between fluoral and hydrogen fluoride, and it is considered that the presence of excess hydrogen fluoride in the system maintains the equilibrium state. As a result, decomposition of the fluoral is suppressed. The fluoral in hydrogen fluoride has been confirmed not only to improve the stability of the compound, but also to raise the boiling point, and the fluoral, which is a low-boiling-point compound, can be easily handled as an adduct of hydrogen fluoride at around room temperature.

In a case of treating the prepared fluoral as a mixture with hydrogen fluoride, an amount of hydrogen fluoride added is usually 0.1 to 100 mol with respect to 1 mol of the prepared fluoral, preferably 1 to 75 mol and more preferably 2 to 50 mol. In a case where the amount of hydrogen fluoride added is 0.1 mol or more, a sufficient stabilizing effect is likely to be obtained. In addition, from the viewpoint of productivity and economy, the amount of hydrogen fluoride added is preferably 100 mol or less.

The mixture of fluoral and hydrogen fluoride may contain excess hydrogen fluoride. Although the presence of excessive hydrogen fluoride may seem undesirable at first sight, the hydrogen fluoride itself may act as an acid catalyst or a dehydrating agent to promote desired reactions. That is, it can be said that there is an advantage in treating fluoral as the mixture with hydrogen fluoride.

The amount of the amine compound represented by General Formula [3] used may be 1 mol or more with respect to 1 mol of the fluoral, but because the reaction proceeds smoothly, it is preferable to use 2 to 10 mol, particularly preferable to use 2 to 5 mol in consideration of a post-treatment operation.

The step of obtaining the aromatic diamine compound represented by General Formula [2] can be performed in the presence of a reaction solvent. Examples of the reaction solvent include an aliphatic hydrocarbon-based solvent, an aromatic hydrocarbon-based solvent, a halogenated hydrocarbon-based solvent, an ether-based solvent, an ester-based solvent, an amide-based solvent, a nitrile-based solvent, and a sulfoxide-based solvent. Specific examples thereof include n-hexane, cyclohexane, n-heptane, benzene, toluene, ethylbenzene, xylene, mesitylene, methylene chloride, chloroform, 1,2-dichloroethane, diethyl ether, tetrahydrofuran, diisopropyl ether, tert-butyl methyl ether, ethyl acetate, n-butyl acetate, N,N-dimethylformamide, N,N-dimethylacetamide, N-methylpyrrolidone, 1,3-dimethyl-2-imidazolidinone, acetonitrile, propionitrile, and dimethylsulfoxide. These reaction solvents can be used alone or in combination. On the other hand, the reaction may be carried out without using a solvent. Performing the reaction without a solvent is preferable from the viewpoint that purification operation after the reaction is simple, and a highly pure target product can be obtained only by a simple purification operation.

In a case where the mixture of fluoral and hydrogen fluoride is reacted with the amine compound, a Lewis acid or a Bronsted acid may be added to the reaction system. As a result, a conversion rate of the condensation reaction can be improved.

The Lewis acid includes metal halides including at least one metal selected from the group consisting of boron (III; oxidation number; hereinafter, the same applies in the present specification), tin (II), tin (IV), titanium (IV), zinc (II), aluminum (III), antimony (III), and antimony (V). As the metal halide to be used, a metal halide having the maximum possible valence is preferable. Among the metal halides, boron (III) trifluoride, aluminum (III) trichloride, zinc (II) dichloride, titanium (IV) tetrachloride, tin (IV) tetrachloride, or antimony (V) pentachloride is particularly preferable. The amount of the Lewis acid used is, for example, 0.001 mol or more, specifically 0.01 to 2.0 mol with respect to 1 mol of the fluoral.

The Bronsted acid is an inorganic acid or an organic acid. Specific examples of the inorganic acid include phosphoric acid, hydrogen chloride, hydrogen bromide, concentrated nitric acid, concentrated sulfuric acid, fuming nitric acid, fuming sulfuric acid, and hydrofluoric acid. Specific examples of the organic acid include formic acid, acetic acid, oxalic acid, benzoic acid, methanesulfonic acid, benzenesulfonic acid, p-toluenesulfonic acid, and trifluoromethanesulfonic acid. The amount of the Bronsted acid used is, for example, 0.001 mol or more, specifically 0.01 to 2.0 mol with respect to 1 mol of the fluoral.

Temperature conditions are, for example, -20°C to +200°C, preferably -10°C to +180°C and more preferably 0°C to +160°C.

Pressure conditions are, for example, atmospheric pressure to 4.0 MPa (absolute pressure; hereinafter, the same applies), preferably atmospheric pressure to 2.0 MPa and more preferably atmospheric pressure to 1.5 MPa.

As a reaction vessel used in this step, a reaction vessel capable of sufficiently carrying out the reaction under normal pressure or under pressure such as metal containers such as stainless steel, Monel^{™}, Hastelloy^{™}, and nickel, and reaction vessels lined with tetrafluoroethylene resin, chlorotrifluoroethylene resin, vinylidene fluoride resin, PFA resin, propylene resin, polyethylene resin, or the like can be used.

The reaction time varies depending on the combination of the mixture of fluoral and hydrogen fluoride and the aryl compound, and the difference in the reaction conditions caused by the amount of Lewis acid and Bronsted acid used as additives. The reaction time is usually within 24 hours. It is preferable to track the progress of the reaction by an analytical unit such as gas chromatography, thin layer chromatography, liquid chromatography, nuclear magnetic resonance, and the like, and to determine the end point of the reaction when the starting material has almost disappeared.

As a post-treatment after the reaction, the reaction-terminated liquid is subjected to a normal purification operation, for example, the reaction-terminated liquid is poured into water or an aqueous solution of an alkali metal inorganic base (for example, sodium hydroxide, potassium hydroxide, sodium hydrogen carbonate, potassium hydrogen carbonate, sodium carbonate, potassium carbonate, and the like) and extracted with an organic solvent (for example, ethyl acetate, toluene, mesitylene, methylene chloride, and the like), whereby a simple substance of the aromatic diamine compound represented by General Formula [2] can be easily obtained. The desired product can be purified to a higher chemical purity product by activated treatment, distillation, recrystallization, column chromatography, and the like, as necessary.

The fluorinated diamine represented by General Formula [1A] or the salt thereof can be obtained by reacting HFA or an equivalent thereof with the aromatic diamine compound represented by General Formula [2].

As HFA or an equivalent thereof, various equivalents such as HFA trihydrate can be used, in addition to HFA gas which is gas at normal temperature and normal pressure. From the viewpoint of reactivity, it is desirable to use the HFA gas which is gas.

The amount of HFA or an equivalent thereof used is typically 0.1 to 10 mol, preferably 1 to 3 mol with respect to 1 mol of the raw material aromatic diamine compound represented by General Formula [2]. By using a sufficiently large amount of HFA or an equivalent thereof, a yield of the target fluorinated diamine represented by General Formula [1A] or a salt thereof can be increased. In addition, by not using an excessive amount of HFA or an equivalent thereof, it is possible to suppress generation of by-products in which excessive -C(CF₃)₂OH groups are introduced.

Examples of the reaction solvent which can be used include an aliphatic hydrocarbon-based solvent, an aromatic hydrocarbon-based solvent, an alcohol-based solvent, a halogenated alcohol-based solvent, a halogenated hydrocarbon-based solvent, an ether-based solvent, an ester-based solvent, an amide-based solvent, a nitrile-based solvent, and a sulfoxide-based solvent. Examples thereof include n-hexane, cyclohexane, n-heptane, benzene, toluene, ethylbenzene, xylene, mesitylene, methanol, ethanol, 2-propanol, trifluoroethanol, hexafluoroisopropanol, methylene chloride, chloroform, 1,2-dichloroethane, diethyl ether, tetrahydrofuran, diisopropyl ether, tert-butyl methyl ether, 1,2-methoxyethane, diglyme, ethyl acetate, n-butyl acetate, N,N-dimethylformamide, N,N-dimethylacetamide, N-methylpyrrolidone, 1,3-dimethyl-2-imidazolidinone, acetonitrile, propionitrile, and dimethylsulfoxide. Among these, fluorous alcohols such as trifluoroethanol and hexafluoroisopropanol are particularly preferable. These reaction solvents can be used alone or in combination. The amount used is 10 to 2000 parts by mass, more preferably 100 to 1000 parts by mass with respect to 100 parts by mass of the raw material aromatic diamine compound represented by General Formula [2].

The use of the Lewis acid or the Bronsted acid can improve a conversion rate of the addition reaction, which is one of preferred aspects.

The Lewis acid includes metal halides including at least one metal selected from the group consisting of boron (III; oxidation number; hereinafter, the same applies in the present specification), tin (II), tin (IV), titanium (IV), zinc (II), aluminum (III), antimony (III), and antimony (V). As the metal halide to be used, a metal halide having the maximum possible valence is preferable. Among these metal halides, boron (III) trifluoride, aluminum (III) trichloride, zinc (II) dichloride, titanium (IV) tetrachloride, tin (IV) tetrachloride, or antimony (V) pentachloride is particularly preferable. The amount of the Lewis acid used is, for example, 0.001 mol or more, preferably 0.01 to 3.0 mol with respect to 1 mol of the raw material aromatic diamine compound represented by General Formula [2] .

The Bronsted acid is an inorganic acid or an organic acid. Specific examples of the inorganic acid include phosphoric acid, hydrogen chloride, hydrogen bromide, concentrated nitric acid, concentrated sulfuric acid, fuming nitric acid, and fuming sulfuric acid. Specific examples of the organic acid include formic acid, acetic acid, oxalic acid, benzoic acid, methanesulfonic acid, benzenesulfonic acid, p-toluenesulfonic acid, and trifluoromethanesulfonic acid. The amount of the Bronsted acid used is, for example, 0.001 mol or more, preferably 0.01 to 3.0 mol with respect to 1 mol of the raw material aromatic diamine compound represented by General Formula [2].

Temperature conditions are usually -20°C to +200°C, preferably 0°C to +180°C and more preferably +20°C to +160°C.

The reaction pressure is usually atmospheric pressure to 4.0 MPa (absolute pressure; hereinafter, the same applies), preferably atmospheric pressure to 3.0 MPa and more preferably atmospheric pressure to 1.5 MPa.

As a reaction vessel, a reaction vessel capable of sufficiently carrying out the reaction under normal pressure or under pressure such as metal containers such as stainless steel, Monel^{™}, Hastelloy^{™}, and nickel, and reaction vessels lined with tetrafluoroethylene resin, chlorotrifluoroethylene resin, vinylidene fluoride resin, PFA resin, propylene resin, polyethylene resin, or the like can be used.

The reaction time varies depending on the amount of the aromatic diamine compound, the reaction solvent, or the acid catalyst used, and the difference in the reaction conditions caused by the HFA equivalent. The reaction time is usually within 24 hours. It is preferable to track the progress of the reaction by an analytical unit such as thin layer chromatography, gas chromatography, liquid chromatography, nuclear magnetic resonance, and the like, and to determine the end point of the reaction when the starting material has almost disappeared.

Post-treatment after the reaction can be carried out by simply distilling off the solvent to easily obtain the intended simple substance of the fluorinated diamine compound represented by General Formula [1A]. The desired product can be purified to a higher chemical purity product by activated treatment, distillation, recrystallization, column chromatography, and the like, as necessary. After the reaction, in a case where it is in a homogeneous state, it can be purified by adding a poor solvent to the solution as it is for recrystallization.

### <Polyamide>

The polyamide according to the present embodiment has a structural unit represented by General Formula [1B].

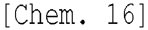

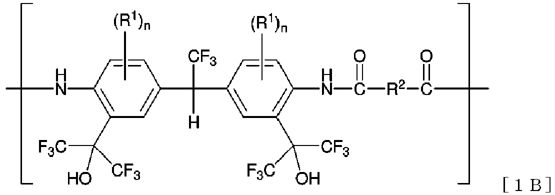

In General Formula [1B],
in a case where a plurality of R¹'s are present, the plurality of R¹'s are each independently at least one selected from the group consisting of an alkyl group, an alkoxy group, a halogen atom, a haloalkyl group, and a haloalkoxy group,
R² is a divalent organic group, and
two n's are each independently an integer of 0 to 3.

Specific aspects of R¹ and n in General Formula [1B] are the same as in General Formula [1A] . Therefore, the description thereof will be omitted.

The polyamide according to the present embodiment tends to exhibit good film-forming properties. It is presumed that this is related to the structure "-CH(CF₃)-" in General Formula [1B].

Introduction of the fluorine atom into a polymer increases solubility in an organic solvent used for film formation. In addition, compared to the "-C(CF₃)₂-" structure common to those skilled in the art in cost and availability, the polyamide according to the present embodiment has poor symmetry. It is presumed that this poorly symmetrical structure results in sparse packing of polymer chains, thereby increasing the solubility in the organic solvent and improving the film-forming properties.

The divalent organic group of R² in General Formula [1B] can include one or more of an aliphatic group, an alicyclic group, an aromatic ring group, a condensed ring group, and the like. In addition, the divalent organic group of R² may include an atom which is neither a carbon atom nor a hydrogen atom, such as an oxygen atom, a nitrogen atom, a sulfur atom, and a halogen atom.

In particular, as the divalent organic group of R², from the viewpoint of heat resistance required for insulations and better dielectric properties, a divalent organic group containing an aromatic ring such as a benzene ring is preferable. More specifically, the divalent organic group of R² can be -Ph-, -Ph-X-Ph-, or the like. Here, Ph is a substituted or unsubstituted phenylene group, X is a single bond or a divalent linking group other than the phenylene group (for example, a linear or branched alkylene group having 1 to 3 carbon atoms, an ether group, a thioether group, a carbonyl group, a sulfone group, a carbonyloxy group, an oxycarbonyl group, and the like).

Particularly preferred examples of R² include the following.

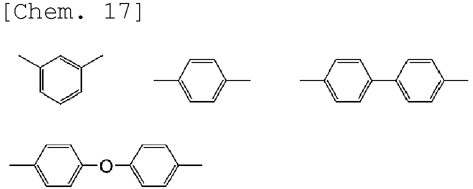

A weight-average molecular weight of the polyamide according to the present embodiment is not particularly limited. However, for example, in a case of being used as an insulating material for producing a high-frequency electronic component or an insulation for a high-frequency electronic component, the weight-average molecular weight of the polyamide is preferably 1,000 or more and 1,000,000 or less, and more preferably 30,000 or more and 500,000 or less. An appropriate weight-average molecular weight can improve, for example, the ease of forming a film on a base material.

The weight-average molecular weight and number-average molecular weight can be measured by gel permeation chromatography (GPC) using polystyrene as a standard substance.

The polyamide according to the present embodiment may have a structural unit different from the structural unit represented by General Formula [1B]. However, as one aspect, from the viewpoint of further improving the performance, preferably 50 to 100 mol%, more preferably 75 to 100 mol%, and still more preferably 90 to 100 mol% of all structural units of the polyamide are the structural unit represented by General Formula [1B]. Substantially all structural units (100%) of the polyamide according to the present embodiment may be the structural unit represented by General Formula [1B].

Preferred examples of the polyamide according to the present embodiment are shown below.

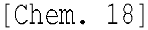

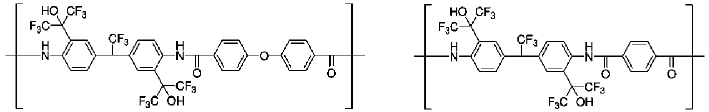

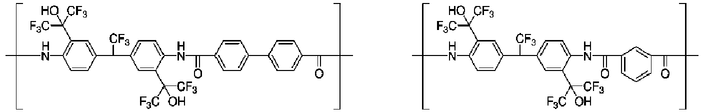

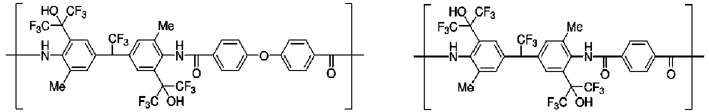

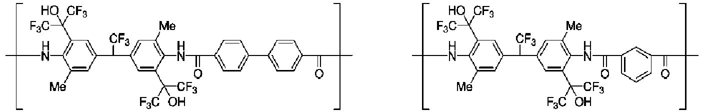

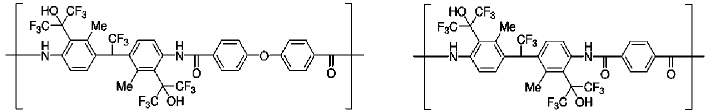

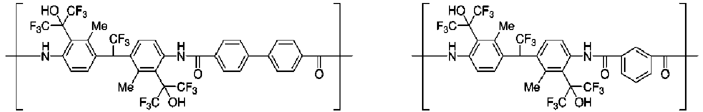

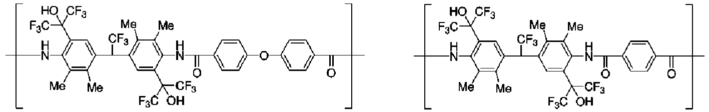

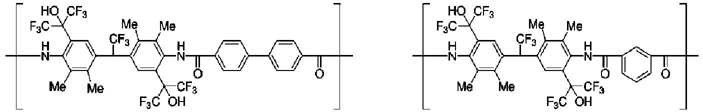

The polyamide according to the present embodiment is preferably used as an insulating material for producing a high-frequency electronic component. Specific applications will be detailed later.

### <Polyamide solution>

The polyamide according to the present embodiment (having the structural unit represented by General Formula [1B]) is usually dissolved in an organic solvent and applied to various uses. One of the preferably applied uses is high-frequency electronic component production applications. That is, a polyamide solution which contains the polyamide according to the present embodiment and an organic solvent is preferably used as an insulating material for producing a high-frequency electronic component.

The organic solvent is preferably at least one selected from the group consisting of an amide-based solvent, an ether-based solvent, an aromatic solvent, a halogen-based solvent, and a lactone-based solvent. These solvents dissolve the polyamide according to the present embodiment well. Specific examples of these solvents include the same organic solvents as those used in the reaction (polycondensation) described in <Method for producing polyamide> below. Incidentally, in a case of preparing the polyamide solution, from the viewpoint of simplification of the production process, it is preferable to use the same organic solvent as the organic solvent used in the reaction (polycondensation).

A concentration of the polyamide solution may be appropriately set according to the application and purpose. From the viewpoint of good film-forming properties, the concentration of the polyamide is preferably 0.1% by mass or more and 50% by mass or less, and more preferably 1% by mass or more and 30% by mass or less.

The polyamide solution according to the present embodiment may contain one or two or more additives in addition to the polyamide and the organic solvent. For example, for the purpose of improving coating properties, leveling properties, film forming properties, storage stability, defoaming properties, and the like, additives such as a surfactant can be used.

Examples of a commercially available product of the surfactant include product name MEGAFACE manufactured by DIC Corporation, product number F142D, F172, F173, or F183; product name Fluorad manufactured by 3M, product number FC-135, FC-170C, FC-430, or FC-431; product name Surflon manufactured by AGC SEIMI CHEMICAL CO., LTD., product number S-112, S-113, S-131, S-141, or S-145; and product name SH-28PA, SH-190, SH-193, SZ-6032, or SF-8428 manufactured by Dow Corning Toray Silicone Co., Ltd. (MEGAFACE is the product name of fluorine-based additives (surfactants or surface modifiers) manufactured by DIC Corporation, Fluorad is the product name of fluorine-based surfactants manufactured by 3M, and Surflon is the product name of fluorine-based surfactants of AGC SEIMI CHEMICAL CO., LTD., each of which is registered as a trademark).

In a case where a surfactant is used, an amount thereof is usually 0.001 to 10 parts by mass with respect to 100 parts by mass of the polyamide.

Incidentally, the polyamide solution according to the present embodiment usually does not contain a photosensitizer such as a quinonediazide compound, or contain a small amount thereof. Specifically, the amount of the photosensitizer in the polyamide solution according to the present embodiment is, for example, 1 part by mass or less, specifically 0.1 parts by mass or less with respect to 100 parts by mass of the polyamide. In a case where the polyamide solution according to the present embodiment is used for applications which do not require patterning with light, no photosensitizer is required. In other words, the polyamide solution according to the present embodiment can be non-photosensitive.

### <Cyclized polyamide>

The cyclized polyamide according to the present embodiment has a structural unit represented by General Formula [1C].

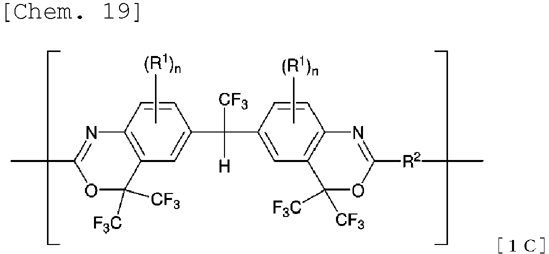

In General Formula [1C],
in a case where a plurality of R¹'s are present, the plurality of R¹'s are each independently at least one selected from the group consisting of an alkyl group, an alkoxy group, a halogen atom, a haloalkyl group, and a haloalkoxy group,
R² is a divalent organic group, and
two n's are each independently an integer of 0 to 3.

Specific aspects of R¹ and n in General Formula [1C] are the same as in General Formula [1A] . Therefore, the description thereof will be omitted.

In addition, specific aspects of R² in General Formula [1C] are the same as in General Formula [1B]. Therefore, the description thereof will be omitted.

A relative permittivity or dielectric loss tangent of the cyclized polyamide according to the present embodiment is small. The reason for this is presumed that the cyclized polyamide according to the present embodiment has a non-planar bulky cyclic skeleton including fluorine, sparse packing of polymer chains is caused by including the asymmetric "-CH(CF₃)-" structure, and the presence of the fluorine atom itself is related.

In addition, the cyclized polyamide according to the present embodiment tends to have high heat resistance. This is presumed to be due to a rigid cyclic skeleton.

The cyclized polyamide according to the present embodiment may have a structural unit different from the structural unit represented by General Formula [1C]. However, as one aspect, from the viewpoint of further improving the performance such as heat resistance, preferably 50 to 100 mol%, more preferably 75 to 100 mol%, and still more preferably 90 to 100 mol% of all structural units of the cyclized polyamide are the structural unit represented by General Formula [1C] . Substantially all structural units (100%) of the cyclized polyamide according to the present embodiment may be the structural unit represented by General Formula [1C].

Preferred examples of the constitutional unit represented by General Formula [1C] are shown below.

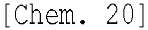

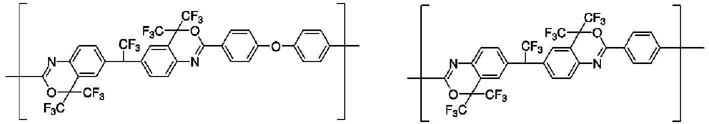

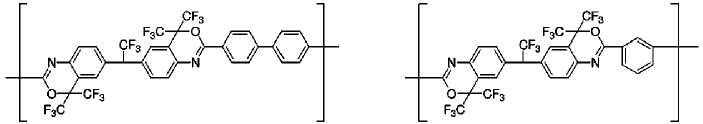

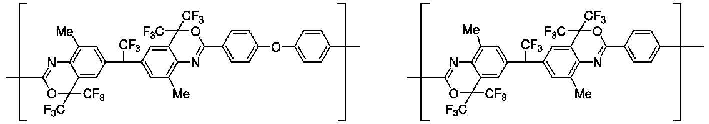

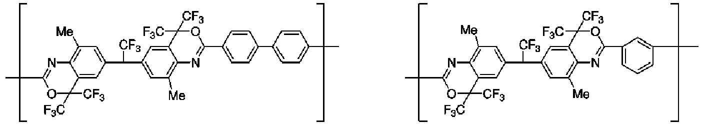

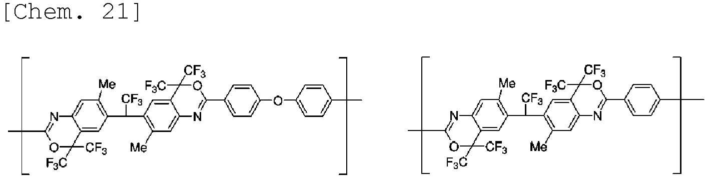

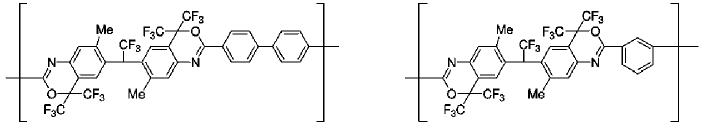

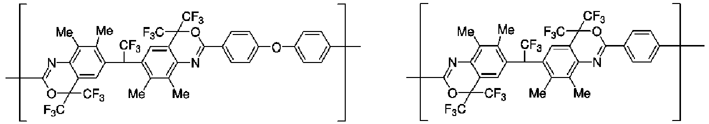

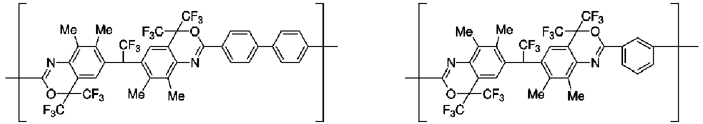

### <Method for producing polyamide>

The polyamide according to the present embodiment (having the structural unit represented by General Formula [1B]) be produced by a reaction (polycondensation) of the fluorinated diamine represented by General Formula [1A] described above or the salt thereof (monomer) with another compound (monomer) . The reaction is usually performed by reacting the fluorinated diamine represented by General Formula [1A] described above or the salt thereof (monomer) with another compound (monomer) in an organic solvent.

From the viewpoint of cost and performance adjustment, the fluorinated diamine represented by General Formula [1A] described above or the salt thereof may be used in combination with another diamine compound. Examples of the diamine compound which can be used include 5-(trifluoromethyl)-1,3-phenylenediamine, 2-(trifluoromethyl)-1,3-phenylenediamine, 4-(trifluoromethyl)-1,3-phenylenediamine, 2-(trifluoromethyl)-1,4-phenylenediamine, 2,2'-bis(trifluoromethyl)benzidine, 2,2'-difluoro-4,4'-diaminodiphenyl, 2,2'-dichloro-4,4'-diaminodiphenyl, 2,2'-dibromo-4,4'-diaminodiphenyl, 2,4-diaminotoluene, 2,5-diaminotoluene, 2,4-dimethyl-1,3-phenylenediamine, 2,5-dimethyl-1,3-phenylenediamine, 2,3-dimethyl-1,4-phenylenediamine, 2,5-dimethyl-1,4-phenylenediamine, 2,6-dimethyl-1,4-phenylenediamine, 2,3,5,6-tetramethyl-1,4-phenylenediamine, 2,2'-dimethylbiphenyl-4,4'-diamine, 2,2'-dimethoxy-4,4'-diaminodiphenyl, 2,2'-diethoxy-4,4'-diaminodiphenyl, 4,4'-diaminodiphenylmethane, 4,4'-diaminodiphenyl ether, 4,4'-diaminodiphenylsulfone, 4,4'-diaminobenzophenone, 1,3-bis(3-aminophenoxy)benzene, 1,3-bis(4-aminophenoxy)benzene, 1,4-bis(4-aminophenoxy)benzene, 4,4'-bis(4-aminophenoxy)biphenyl, bis(4-(3-aminophenoxy)phenyl)sulfone, bis(4-(4-aminophenoxy)phenyl)sulfone, 2,2-bis(4-(4-aminophenoxy)phenyl)propane, 2,2-bis(4-(4-aminophenoxy)phenyl)hexafluoropropane, 2,2-bis(4-(3-aminophenoxy)phenyl)propane, 2,2-bis(4-(3-aminophenoxy)phenyl)hexafluoropropane, 2,2-bis(4-(4-aminophenyl)hexafluoropropane, 2,2-bis(4-aminophenyl)hexafluoropropane, 2,2-bis(3-aminophenyl)hexafluoropropane, 2,2-bis(3-amino-4-hydroxyphenyl)hexafluoropropane, 2,2-bis(3-amino-4-methylphenyl)hexafluoropropane, and 4,4'-diaminobenzanilide.

Examples of the diamine which can be used in combination also include diamines which have a -C(CF₃)₂-OH group (a hexafluoroisopropanol group) but do not correspond to General Formula [1A].

Specific examples thereof include diamines having a -C(CF₃)₂-OH group, described in Japanese Unexamined Patent Publication No. 2007-119503, Japanese Unexamined Patent Publication No. 2007-119504, Japanese Unexamined Patent Publication No. 2008-150534, Japanese Unexamined Patent Publication No. 2014-125455, Japanese Unexamined Patent Publication No. 2014-129340, and the like. Among these, diamines as shown below are preferable.

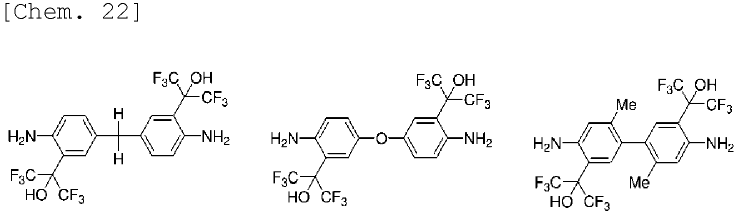

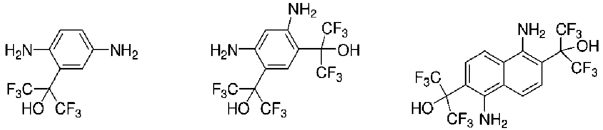

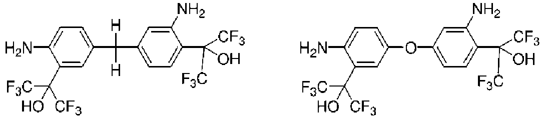

Incidentally, from the viewpoint of obtaining sufficient performance, it is preferable that 30 mol% or more of all diamine compounds used in the production of the polyamide are the fluorinated diamine represented by General Formula [1A] or the salt thereof.

In the production of the polyamide, (i) only one kind of the fluorinated diamine represented by General Formula [1A] or the salt thereof may be used; (ii) two or more kinds of the fluorinated diamine represented by General Formula [1A] or the salt thereof may be used in combination; or (iii) one kind or two or more kinds of the fluorinated diamine represented by General Formula [1A] or the salt thereof and one kind or two or more kinds of the diamine compound not corresponding to General Formula [1A] may be used in combination.

Preferred examples of "another compound (monomer) " to be reacted with the fluorinated diamine or the salt thereof (monomer) include dicarboxylic acid or a derivative thereof (diester, dicarboxylic acid halide, active ester compound, and the like). Preferred examples of the dicarboxylic acid or a derivative thereof include a compound represented by General Formula [DC-1] or [DC-2].

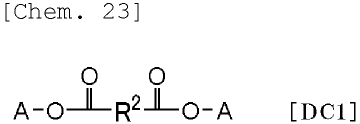

In General Formula [DC1], R² is the same as R² in General Formula [1B], and two A's each independently represent a hydrogen atom, an alkyl group having 1 to 10 carbon atoms, or an aromatic hydrocarbon group having 6 to 10 carbon atoms.

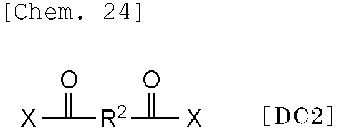

In General Formula [DC2], R² is the same as R² in General Formula [1B], and two X's each independently represent a fluorine atom, a chlorine atom, a bromine atom, an iodine atom, or an active ester group.

A compound in which X is an "active ester group" is obtained, for example, by reacting a dicarboxylic acid and an active esterification agent in the presence of a dehydration condensation agent. Specific examples of a preferred dehydration condensation agent include dicyclohexylcarbodiimide, 1-ethoxycarbonyl-2-ethoxy-1,2-dihydroquinoline, 1,1'-carbonyldioxy-di-1,2,3-benzotriazole, and N,N'-disuccinimidyl carbonate. Examples of a preferred active esterification agent include N-hydroxysuccinimide, 1-hydroxybenzotriazole, N-hydroxy-5-norbornene-2,3-dicarboxylic imide, ethyl 2-hydroxyimino-2-cyanoacetate, and 2-hydroxyimino-2-cyanoacetic acid amide.

Specific examples of the dicarboxylic acid itself or a dicarboxylic acid from which the dicarboxylic acid derivative is aliphatic dicarboxylic acids such as derived oxalic acid, malonic acid, succinic acid, glutaric acid, adipic acid, pimelic acid, suberic acid, azelaic acid, sebacic acid; aromatic dicarboxylic acids such as phthalic acid, isophthalic acid, terephthalic acid, 3,3'-dicarboxylic diphenyl ether, 3,4-dicarboxylic diphenyl ether, 4,4'-dicarboxylic diphenyl ether, 3,3'-dicarboxylic diphenylmethane, 3,4-dicarboxylic diphenylmethane, 4,4'-dicarboxylic diphenylmethane, 3,3'-dicarboxyldiphenyldifluoromethane, 3,4-dicarboxyldiphenyldifluoromethane, 4,4'-dicarboxyldiphenyldifluoromethane, 3,3'-dicarboxyldiphenylsulfone, 3,4-dicarboxyldiphenylsulfone, 4,4'-dicarboxyldiphenylsulfone, 3,3'-dicarboxyldiphenylsulfide, 3,4-dicarboxyldiphenylsulfide, 4,4'-dicarboxyldiphenylsulfide, 3,3'-dicarboxylic diphenyl ketone, 3,4-dicarboxylic diphenyl ketone, 4,4'-dicarboxylic diphenyl ketone, 3,3'-dicarboxylic diphenylmethane, 3,4-dicarboxyldiphenylmethane, 4,4'-dicarboxyldiphenylmethane, 2,2-bis(3-carboxyphenyl)propane, 2,2-bis(3,4'-carboxyphenyl)propane, 2,2-bis(4-carboxyphenyl)propane, 2,2-bis(3-carboxyphenyl)hexafluoropropane, 2,2-bis(3,4'-carboxyphenyl)hexafluoropropane, 2,2-bis(4-carboxyphenyl)hexafluoropropane, 1, 3-bis(3-carboxyphenoxy)benzene, 1,4-bis(3-carboxyphenoxy)benzene, 1,4-bis(4-carboxyphenoxy)benzene, 3,3'-(1,4-phenylenebis(1-methylethylidene))bisbenzoic acid, 3,4'-(1,4-phenylenebis(1-methylethylidene))bisbenzoic acid, 4,4'-(1,4-phenylenebis(1-methylethylidene))bisbenzoic acid, 2,2-bis(4-(3-carboxyphenoxy)phenyl)propane, 2,2-bis(4-(4-carboxyphenoxy)phenyl)propane, 2,2-bis(4-(3-carboxyphenoxy) phenyl )hexafluoropropane, 2,2-bis(4-(4-carboxyphenoxy)phenyl)hexafluoropropane, 2,2-bis(4-(3-carboxyphenoxy)phenyl)sulfide, 2,2-bis(4-(4-carboxyphenoxy)phenyl)sulfide, 2,2-bis(4-(3-carboxyphenoxy)phenyl)sulfone, and 2,2-bis(4-(4-carboxyphenoxy)phenyl)sulfone; perfluorononenyloxy group-containing dicarboxylic acids such as 4-(perfluorononenyloxy)phthalic acid, 5-(perfluorononenyloxy)isophthalic acid, 2-(perfluorononenyloxy)terephthalic acid, and 4-methoxy-5-(perfluorononenyloxy)isophthalic acid; and perfluorohexenyloxy group-containing dicarboxylic acids such as 4-(perfluorohexenyloxy)phthalic acid, 5-(perfluorohexenyloxy)isophthalic acid, 2-(perfluorohexenyloxy)terephthalic acid, and 4-methoxy-5-(perfluorohexenyloxy) isophthalic acid.

The dicarboxylic acid or the derivative thereof may be used alone, or two or more thereof may be used in combination.

Preferred examples of the dicarboxylic acid or the derivative of the dicarboxylic acid include aromatic dicarboxylic acids and derivatives thereof. Particularly preferred examples of the dicarboxylic acid or the derivative of the dicarboxylic acid include the following. In the following, definition and specific aspects of A are the same as those of General Formula [DC-1], and definition and specific aspects of X are the same as those of General Formula [DC-2].

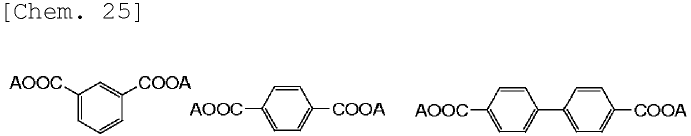

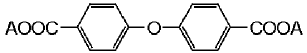

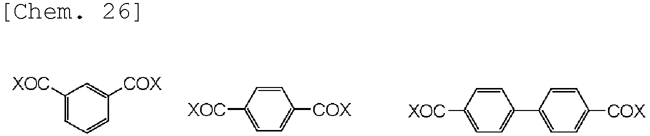

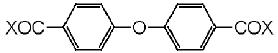

As the organic solvent used for the reaction (polycondensation), a solvent in which the raw material compounds are dissolved can be used without particular limitation. Specific examples thereof include an amide-based solvent, an ether-based solvent, an aromatic solvent, a halogen-based solvent, and a lactone-based solvent. More specific examples thereof include:, as the amide-based solvent, N,N-dimethylformamide, N,N-dimethylacetamide, N-methylformamide, hexamethylphosphoric triamide, or N-methyl-2-pyrrolidone; as the ether-based solvent, diethyl ether, dipropyl ether, diisopropyl ether, dibutyl ether, cyclopentyl methyl ether, diphenyl ether, dimethoxyethane, diethoxyethane, tetrahydrofuran, dioxane, or trioxane; as the aromatic solvent, benzene, anisole, nitrobenzene, or benzonitrile; as the halogen-based solvent, chloroform, dichloromethane, 1,2-dichloroethane, or 1,1,2,2-tetrachloroethane; and as the lactone-based solvent, γ-butyrolactone, γ-valerolactone, ε-valerolactone, γ-caprolactone, ε-caprolactone, or α-methyl-γ-butyrolactone. Only one type of organic solvent may be used, or two or more types thereof may be mixed and used.

The temperature during the reaction may be appropriately set, for example, between -100°C to 100°C. In addition, the reaction may be carried out in an inert gas environment such as nitrogen and argon.

In the production of polyamide, terminal modification with an addition-reactive group, which is often carried out in known polyamide resins, may be carried out.

The addition-reactive group is not particularly limited as long as it is a group which undergoes an addition polymerization reaction (curing reaction) by heating. Any reactive group selected from the group consisting of a group including an acetylene bond, such as a phenylethynyl group, a nadic acid group, and a maleimide group is preferable, a group including an acetylene bond, such as a phenylethynyl group, is more preferable, and a phenylethynyl group is still more preferable.

The addition-reactive group is introduced into the terminal of polymer by reacting a compound having the addition-reactive group and an acid anhydride group or an amino group in one molecule with an amino group or an acid anhydride group of the terminal of polymer. This reaction is preferably a reaction forming an imide ring. Examples of the compound having an acid anhydride group or an amino group together with the addition-reactive group in the molecule include 4-(2-phenylethynyl) phthalic anhydride, phenylethynyl trimellitic anhydride, 4-(2-phenylethynyl) aniline, 4-ethynyl-phthalic anhydride, 4-ethynylaniline, nadic acid anhydride, and maleic acid anhydride.

In addition, in the production of polyamide, the terminal may be capped with dicarboxylic acid anhydrides such as maleic acid anhydride, phthalic acid anhydride, nadic acid anhydride, ethynyl phthalic acid anhydride, and hydroxy phthalic acid anhydride, hydroxyaniline, aminobenzoic acid, dihydroxyaniline, carboxyhydroxyaniline, and dicarboxyaniline.

After completion of the polycondensation reaction, for the purpose of removing residual monomers and low-molecular-weight substances, it is preferable to add the reaction solution to a poor solvent (for example, water, alcohol, and the like) to precipitate, isolate, and purify the polyamide. As a result, polyamides with reduced impurities can be produced, and thus insulation with better dielectric properties can be obtained.

The polyamide with reduced impurities may be dissolved again in the organic solvent. By doing so, it is possible to obtain a polyamide solution with a small amount of impurities.

### <Method for producing cyclized polyamide>

Using the polyamide produced as described above, the cyclized polyamide can be produced.

Specifically, by a method including
· a first step of producing a polyamide by <Method for producing polyamide> described above, and
· a second step of dehydrating and ring-closing the polyamide obtained in the first step,
   the cyclized polyamide according to the present embodiment (polymer having the structural unit represented by General Formula [1C] described above) can be produced.

The dehydration and ring-closing reaction in the second step is usually carried out by heating. Specifically, the polyamide obtained in the first step is heated to 100°C or higher and 400°C or lower. As a result, cyclodehydration proceeds in the polyamide. Then, the cyclized polyamide according to the present embodiment can be obtained.

The heating of the polyamide may be carried out by heating the polyamide solution or by heating the polyamide in a solid form (for example, in a form of a film), preferably the latter. This will be described later in detail as a method for producing an insulation for a high-frequency electronic component.

### <Method for producing insulation for high-frequency electronic component and insulation for high-frequency electronic component>

In the present embodiment, an insulation for a high-frequency electronic component, which includes the cyclized polyamide, can typically be obtained by heating the above-described polyamide or its solution.

Specifically, the insulation for a high-frequency electronic component, which includes the cyclized polyamide having the structural unit represented by General Formula [1C], can be produced through each of the following steps. Incidentally, the following drying step and heating step may be performed continuously.
· a step (coating step) of applying the above-described polyamide solution onto a supporting base material
· a step (drying step) of drying the solvent contained in the applied polyamide solution to obtain a resin film including the polyamide
· a step (heating step) of heat-treating the obtained resin film to form a cured film

Each of the above-described steps will be explained below.

### (Coating step)

A coating method in the coating step is not particularly limited, and a known method can be adopted. Depending on coating film thickness, solution viscosity, and the like, a known coating devices such as spin coater, bar coater, doctor blade coater, air knife coater, roll coater, rotary coater, flow coater, die coater, and lip coater can be appropriately used.

The supporting base material is not particularly limited, but an inorganic base material or an organic base material is suitable. Specific examples thereof include glass, a silicon wafer, stainless steel, alumina, copper, nickel, polyethylene terephthalate, polyethylene glycol terephthalate, polyethylene glycol naphthalate, polycarbonate, polyimide, polyamide-imide, polyetherimide, polyetheretherketone, polypropylene, polyether sulfone, polyethylene terephthalate, polyphenylene sulfone, and polyphenylene sulfide.

Among these, from the viewpoint of heat resistance, it is preferable to use an inorganic base material, and it is more preferable to use an inorganic base material such as glass, silicon wafer, and stainless steel.

By adjusting the coating amount per unit area in the coating step and the concentration of the polyamide solution used for coating, a thickness of the finally obtained film (cured film and the insulation for a high-frequency electronic component) can be adjusted.

The thickness of the finally obtained film (cured film and the insulation for a high-frequency electronic component) is usually 1 um or more and 1000 um or less, preferably 5 um or more and 500 um or less. In a case where the thickness is 1 um or more, strength of the film itself can be sufficient. In a case where the thickness is 1000 um or less, defects such as cissing, dents, and cracks can be easily suppressed.

### (Drying step)

In the drying step, the solvent in the applied polyamide solution is generally volatilized by heating using a hot plate. Depending on the type of solvent in which the polyamide is dissolved, the heating temperature in the drying step is preferably 50°C or higher and 250°C or lower, and more preferably 80°C or higher and 200°C or lower. The heating temperature in the drying step is usually lower than a temperature in the subsequent heating step.

In a case where the heating temperature in the drying step is 50°C or higher, the drying can be sufficiently easily performed. In addition, in a case where the heating temperature in the drying step is 250°C or lower, defects such as cissing, dents, and cracks due to rapid solvent evaporation can be suppressed, and a uniform film can be easily formed.

### (Heating step)

In the heating step, the resin film obtained in the drying step is cured by a heat treatment at a high temperature. By heating, the ring-closing reaction of the polyamide in the resin film proceeds, and an insulation (cured film) for a high-frequency electronic component including the cyclized polyamide can be obtained. In the heating step, it is expected to remove residual solvent which cannot be removed in the drying step, improve cyclization rate, and improve physical properties. The temperature of the heating step is preferably 100°C or higher and 400°C or lower, and more preferably 150°C or higher and 350°C or lower. In a case where the temperature of the heating step is 100°C or higher, the cyclization reaction can be sufficiently progressed easily. In addition, in a case where the temperature of the heating step is 400°C or lower, it is easy to suppress occurrence of defects such as cracks.

The heating step is preferably performed using a device such as an inert gas oven, a hot plate, a box-type dryer, and a conveyor-type dryer, but is not limited to these devices. From the viewpoint of preventing oxidation of the resin film, and removing the residual solvent, the heating step is preferably performed under an inert gas stream. Examples of the inert gas include nitrogen and argon. A flow rate of the inert gas is desirably 1 L/min or more and 5 L/min or less. In a case where the flow rate of the inert gas is 1 L/min or more, it is easy to sufficiently remove the solvent and cure the resin film. In addition, in a case where the flow rate of the inert gas is 5 L/min or less, the entire resin film dries and cures uniformly, and defects such as cracks are less likely to occur.

Depending on the application and purpose, a peeling step of peeling off the cured film (containing the cyclized polyamide) from the supporting base material after the heating step and using it as a cyclized polyamide substrate may be performed. The peeling step can be performed after cooling from room temperature (20°C) to approximately 400°C after the heating step. A peeling agent may be applied onto the supporting base material in advance in order to facilitate the peeling. The peeling agent used in this case is not particularly limited, and examples thereof include silicon-based or fluorine-based release agents.

The insulation for a high-frequency electronic component (hereinafter, also simply referred to as an "insulation") according to the present embodiment includes the cyclized polyamide having the structural unit represented by General Formula [1C] described above. The insulation according to the present embodiment may additionally include the polyamide having the structure represented by General Formula [1B] described above.

The insulation according to the present embodiment is typically film-like. A film-like insulation can be obtained, for example, by using the polyamide solution and going through the coating step, the drying step, and the heating step, as described above.

For convenience of application to the manufacturing process of electronic devices, it is preferable that the insulation according to the present embodiment has good heat resistance. A 5%-weight-loss temperature (Td₅) can be used as an index for the heat resistance. As described in Examples later, Td₅ can be quantified by using a differential scanning calorimeter and reading data in a case where the temperature of the insulation is increased at a constant rate.

Td₅ is preferably 350°C or higher, more preferably 380°C or higher, and still more preferably 400°C or higher. Although there is no particular upper limit for Td₅, from the viewpoint of realistic polymer design, the upper limit of Td₅ is, for example, 600°C.

Since the insulation according to the present embodiment includes the cyclized polyamide having the structural unit represented by General Formula [1C], the insulation according to the present embodiment is preferably used as an insulation provided in a high-frequency equipment (communication equipment and the like) used in 5G (fifth generation mobile communication system).

Specifically, a dielectric loss tangent of the insulation according to the present embodiment at a frequency of 28 GHz is preferably 0.012 or less and more preferably 0.007 or less. The lower limit value of the dielectric loss tangent is ideally 0, but is practically approximately 0.0002.

In addition, a relative permittivity of the insulation according to the present embodiment at a frequency of 28 GHz is preferably 3.1 or less and more preferably 2.8 or less. The lower limit value of the relative permittivity is practically 2.0.

By designing the insulation so that the dielectric loss tangent at a frequency of 28 GHz is 3.1 or less and/or the relative permittivity at a frequency of 28 GHz is 0.012 or less, it is possible to sufficiently increase transmission speed and reduce transmission loss in 5G.

### <High-frequency electronic component and high-frequency appliance>

The high-frequency electronic component according to the present embodiment includes the above-described insulation. In addition, by using the high-frequency electronic component, a high-frequency appliance (communication terminal and the like) can be produced.

As an example, the high-frequency electronic component according to the present embodiment can be obtained by providing wiring portions on one or both sides of the film-like insulation. By doing so, the transmission speed can be increased and/or the transmission loss can be reduced.

In addition, the above-described insulation may have good heat resistance. Therefore, even in a case where the temperature of the insulation is likely to rise (for example, drying, vapor deposition, plasma treatment, and the like) in the process of producing the high-frequency electronic component, the performance of the insulation is unlikely to change. This is preferable for the production of electronic parts.

Examples of a method for forming a wiring portion on the film-like insulation include a method in which the wiring portion is formed by forming a conductive layer consisting of a conductive material such as copper, indium tin oxide (ITO), polythiophene, polyaniline, and polypyrrole by a lamination method, a metallizing method, a sputtering method, a vapor deposition method, a coating method, or a printing method, and then patterning the conductive layer. Before forming the conductive layer, in order to improve adhesion force between the insulation and the conductive layer, a surface of the film-like insulation may be modified by a plasma treatment or the like. In addition, an adhesive may be used to improve the adhesion force.

The embodiments of the present invention have been described above, but these are examples of the present invention and various configurations other than the above can be adopted. In addition, the present invention is not limited to the above-described embodiments, and modifications, improvements, and the like within the range in which the object of the present invention can be achieved are included in the present invention.

### [Examples]

Embodiments of the present invention will be described in detail based on Examples and Comparative Examples. It should be noted that the present invention is not limited to Examples only.

Hereinafter, "%" of a composition analysis value represents the "area%" of a composition, which is obtained by measuring the raw material or product by gas chromatography (hereinafter, referred to as GC; unless otherwise specified, the detector is FID) or liquid chromatography (hereinafter, referred to as LC; unless otherwise specified, the detector is UV).

### <Various measurements and evaluation methods>

First, various measurements and evaluation methods will be explained.

### [Weight-average molecular weight (Mw) and number-average molecular weight (Mn)]

A weight-average molecular weight and a number-average molecular weight were measured using gel permeation chromatography (GPC, HLC-8320 manufactured by TOSOH CORPORATION) with polystyrene as a standard substance. Tetrahydrofuran (THF) was used as the mobile phase, and TSKgel SuperHZM-H was used as the column.

### [Infrared absorption spectrum (IR) measurement]

The infrared absorption spectrum of a compound or a film was measured using Nicolet NEXUS470FT-IR (manufactured by ThermoFisher Scientific).

### [Heat resistance]

For the 5%-weight-loss temperature (Td₅), with a simultaneous differential thermal thermogravimetric measurement device (manufactured by Hitachi High-Tech Science Corporation, model name: STA7200), the measurement was performed under the conditions of a temperature increase rate of 10 °C/min from the initial temperature of 30°C.

[Dielectric properties] (relative permittivity (εr) and dielectric loss tangent (tan δ))

Relative permittivity (εr) and dielectric loss tangent (tan δ) of cured films obtained in Examples below or films of Comparative Examples at a frequency condition of 28 GHz, a temperature of 23°C, and a relative humidity of 50 %RH were measured by a split cylinder resonator method. As a measurement device, a network analyzer device name "N5290A" manufactured by Keysight Technologies and a split cylinder resonator (28 GHz CR-728) manufactured by KANTO Electronic Application and Development Inc. were used.

### [Curing temperature]

Using a differential scanning calorimeter (manufactured by SII NanoTechnology Inc., model name: X-DSC7000), measurement was performed under the conditions of an initial temperature of 30°C, a measurement temperature range of -40°C to 350°C, and a temperature increase rate of 10 °C/min. Specifically, the temperature at which the maximum value of the endothermic peak generated in a case where the polyamide is ring-closed and converted to the cyclized polyamide was taken as the curing temperature.

### <Synthesis of fluorinated diamine>

### [Catalyst Preparation Example]

896 g of special grade reagent CrCl₃·6H₂O was dissolved in pure water to be 3.0 L. 400 g of granular alumina was immersed in this solution and left for a whole day and night. Next, the solution was filtered to take out the alumina, kept at 100°C in a hot air circulating dryer, and dried for a whole day and night.

The obtained chromium-supported alumina was filled in a cylindrical SUS316L reaction tube with a diameter of 4.2 cm and a length of 60 cm, equipped with an electric furnace. The temperature was raised to 300°C while flowing nitrogen gas into the reaction tube at a flow rate of approximately 20 mL/min. In a case where outflow of water was no longer observed, hydrogen fluoride was entrained in the nitrogen gas and its concentration was gradually increased. In a case where a hot spot due to fluorination of the packed chromium-supported alumina reached the outlet end of the reaction tube, the reactor temperature was raised to 350°C and held there for 5 hours. A catalyst was thus prepared.

### (Preparation Example 1: Preparation of fluoral represented by following scheme)

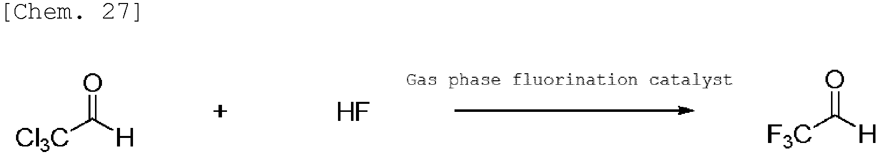

A gas phase reactor (made of SUS316L, diameter: 2.5 cm, length: 40 cm) consisting of a cylindrical reaction tube equipped with an electric furnace was filled with 125 mL of the catalyst prepared in the above-described catalyst preparation example.

The temperature of the reaction tube was raised to 280°C while flowing air at a flow rate of approximately 100 mL/min into the reaction tube filled with the catalyst, and hydrogen fluoride was introduced at a rate of approximately 0.32 g/min over 1 hour. Next, chloral (trichloroethanal) as a raw material was started to be supplied to the reaction tube at a rate of approximately 0.38 g/min (contact time: 15 seconds) . One hour after the start of the reaction, the reaction was stabilized, so that gas flowing out of the reactor was collected over 54 hours in a cylinder made of SUS304 with a blowing pipe cooled with a coolant of -15°C.

In a case where hydrogen fluoride content, hydrogen chloride content, and organic matter content were calculated by titration for 1454.4 g of the obtained collected liquid containing fluoral, hydrogen fluoride was 40% by weight, hydrogen chloride was 11% by weight, the content of the organic matter was 49% by weight, and a recovery rate of the organic matter was 88% (based on moles of feed chloral raw material). In addition, in a case where a part of the recovered organic matter was collected in a resin NMR tube, and the degree of fluorination was confirmed by ¹⁹F-NMR, it was confirmed that substantially no low-order fluorinated compounds were detected and fluorination was progressing quantitatively.

Next, 1300 g (hydrogen fluoride: 40% by weight, hydrogen chloride: 11% by weight, organic matter: 49% by weight) of a part of the collected mixture containing fluoral was charged into a 2000 mL SUS304 reactor equipped with a cooling pipe through which a coolant of -15°C was passed, a thermometer and a stirrer, and the reactor was heated to 25°C. Hydrogen chloride passing through a top tower of the cooling pipe was removed by being absorbed by water, while hydrogen fluoride was refluxed in the cooling pipe under normal pressure. After 10 hours of reflux, the reactor was sampled, and hydrogen fluoride content, hydrogen chloride content, and organic matter content were calculated by titration on the mixture to be hydrogen fluoride: 44% by weight, hydrogen chloride: 1% by weight, and organic matter: 55% by weight. In addition, in a case where a part of the mixture was collected in a resin NMR tube, from an integral ratio of ¹⁹F-NMR, it was confirmed that fluoral in anhydrous hydrogen fluoride was converted to 1,2,2,2-tetrafluoroethanol which is a composition of fluoral and hydrogen fluoride. On the other hand, in a case where the water used to absorb the hydrogen chloride was subjected to titration, although hydrogen fluoride was partially included due to entrainment of droplets, almost no organic matter was included.

### [NMR data]

1,2,2,2-tetrafluoroethanol:
¹⁹F-NMR (400 MHz, CFCl₃) δ (ppm) : -85.8 (3F, s), -137.8 (1F, d, J = 54.9 Hz)

Hydrogen fluoride:
¹⁹F-NMR (400 MHz, CFCl₃) δ (ppm) : -193.4 (1F, s)

### (Preparation Example 2: Synthesis of aromatic diamine compound represented by following scheme)

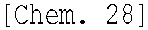

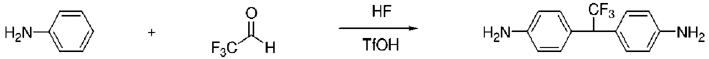

In a 1 L stainless steel autoclave reactor equipped with a pressure gauge, a thermometer protection tube, an insert tube, and a stirring motor, 205 g (fluoral: 1.15 mol, hydrogen fluoride: 4.51 mol) of the fluoral-containing mixture obtained in Preparation Example 1 (hydrogen fluoride: 44% by weight, hydrogen chloride: 1% by weight, organic matter: 55% by weight), 186 g (9.29 mol) of hydrogen fluoride, and 321 g (3.45 mmol) of aniline were weighed, and the mixture was heated in a 90°C oil bath and reacted at an absolute pressure of 0.05 MPa for 7 hours. The reaction solution was poured into a mixture of 600 g of ice water and 1080 g of toluene, and 1250 g of 48% potassium hydroxide aqueous solution was added dropwise thereto for neutralization. After the organic layer recovered by the liquid separation operation was further washed with 750 g of 18% potassium hydroxide aqueous solution and then with 600 g of clean water, the organic layer was heated to 80°C, and 410 g of n-heptane was added dropwise thereto for recrystallization. The precipitated solid was filtered off to obtain the target product
1,1,1-trifluoro-2,2-bis (4-aminophenyl) ethane in an amount of 189 g, yield of 63%, and purity of 99.6% (GC).

### [NMR data]

1,1,1-trifluoro-2,2-bis(4-aminophenyl)ethane:
¹H-NMR (400 MHz, CDCl₃) δ (ppm) : 3.42 (4H, s), 4.45 (1H, q, J = 10.1 Hz), 6.62 (4H, d, J = 8.3 Hz), 7.12 (4H, d, J = 8.3 Hz)
¹⁹F-NMR (400 MHz, CDCl₃, CFCl₃) δ (ppm) : -66.9 (3F, d, J = 11.5 Hz)

### (Preparation Example 3: Synthesis of aromatic diamine compound represented by following scheme)

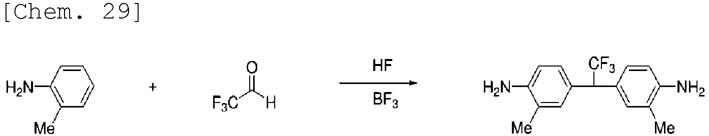

In a 1 L stainless steel autoclave reactor equipped with a pressure gauge, a thermometer protection tube, an insert tube, and a stirring motor, 196 g (fluoral: 1.10 mol, hydrogen fluoride: 4.31 mol) of the fluoral-containing mixture obtained in Preparation Example 1 (hydrogen fluoride: 44% by weight, hydrogen chloride: 1% by weight, organic matter: 55% by weight), 178 g (8.89 mol) of hydrogen fluoride, and 353 g (3.30 mmol) of o-toluidine were weighed, and the mixture was heated in a 90°C oil bath and reacted at an absolute pressure of 0.05 MPa for 7 hours. The reaction solution was poured into a mixture of 600 g of ice water and 1080 g of toluene, and 1250 g of 48% potassium hydroxide aqueous solution was added dropwise thereto for neutralization. After the organic layer recovered by the liquid separation operation was further washed with 750 g of 18% potassium hydroxide aqueous solution and then with 600 g of clean water, the organic layer was heated to 80°C, and 410 g of n-heptane was added dropwise thereto for recrystallization. The precipitated solid was filtered off to obtain the target product 1,1,1-trifluoro-2,2-bis(3-methyl-4-aminophenyl)ethane in an amount of 252 g, yield of 86%, and purity of 99.4% (GC).

### [NMR data]

1,1,1-trifluoro-2,2-bis(3-methyl-4-aminophenyl)ethane:
¹H-NMR (400 MHz, CDCl₃) δ (ppm): 2.13 (6H, s), 3.14 (4H, s), 4.41 (1H, q, J = 10.4 Hz), 6.62 (2H, d, J = 10.4 Hz), 7.01 (2H, s), 7.02 (2H, d, J = 8.3 Hz)
¹⁹F-NMR (400 MHz, CDCl₃) δ (ppm) : -66.7 (3F, d, J = 11.5 Hz)

### (Preparation Example 4: Synthesis of aromatic diamine compound represented by following scheme)

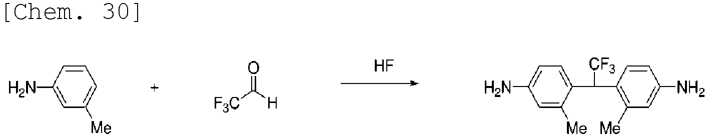

In a 1 L stainless steel autoclave reactor equipped with a pressure gauge, a thermometer protection tube, an insert tube, and a stirring motor, 209 g (fluoral: 1.17 mol, hydrogen fluoride: 4.60 mol) of the fluoral-containing mixture obtained in Preparation Example 1 (hydrogen fluoride: 44% by weight, hydrogen chloride: 1% by weight, organic matter: 55% by weight), 189 g (9.44 mol) of hydrogen fluoride, and 300 g (2.80 mmol) of m-toluidine were weighed, and the mixture was heated in a 150°C oil bath and reacted at an absolute pressure of 0.45 MPa for 8 hours. The reaction solution was poured into a mixture of 1030 g of ice water, 690 g of toluene, and 500 g of ethyl acetate, and 1720 g of 48% potassium hydroxide aqueous solution was added dropwise thereto for neutralization. The organic layer recovered by the liquid separation operation was washed with 1000 g of clean water, and then concentrated to approximately 400 g by an evaporator. The oily crude product was recrystallized from 350 g of ethanol and 560 g of methylcyclohexane to obtain the target product 1,1,1-trifluoro-2,2-bis(2-methyl-4-aminophenyl)ethane in an amount of 207 g, yield of 60%, and purity of 98.4% (GC).

### (Preparation Example 5: Synthesis of aromatic diamine compound represented by following scheme)

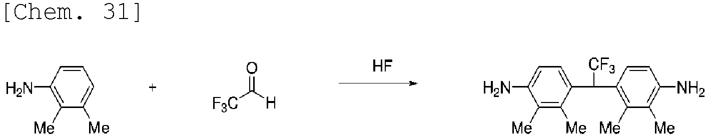

In a 1 L stainless steel autoclave reactor equipped with a pressure gauge, a thermometer protection tube, an insert tube, and a stirring motor, 196 g (fluoral: 1.10 mol, hydrogen fluoride: 4.31 mol) of the fluoral-containing mixture obtained in Preparation Example 1 (hydrogen fluoride: 44% by weight, hydrogen chloride: 1% by weight, organic matter: 55% by weight), 178 g (8.89 mol) of hydrogen fluoride, and 267 g (2.20 mmol) of 2,3-dimethylaniline were weighed, and the mixture was heated in a 150°C oil bath and reacted at an absolute pressure of 1.2 MPa for 19 hours. The reaction solution was poured into a mixture of 700 g of ice water and 1400 g of ethyl acetate, and 1570 g of 48% potassium hydroxide aqueous solution was added dropwise thereto for neutralization. The organic layer recovered by the liquid separation operation was further washed with 600 g of clean water, and then concentrated to approximately 800 g by an evaporator. The concentrated solution was heated to 60°C, and 710 g of n-heptane was added dropwise thereto for recrystallization. The precipitated solid was filtered off to obtain the target product 1,1,1-trifluoro-2,2-bis(2,3-dimethyl-4-aminophenyl)ethane in an amount of 168 g, yield of 47%, and purity of 99.1% (GC).

### [NMR data]

1,1,1-trifluoro-2,2-bis(2,3-dimethyl-4-aminophenyl)ethane:
¹H-NMR (400 MHz, CDCl₃) δ (ppm): 2.10 (6H, s), 2.18 (6H, s), 4.19 (4H, bs), 5.01 (1H, q, J = 9.6 Hz), 6.59 (2H, d, J = 8.4 Hz), 7.07 (2H, d, J = 8.4 Hz)
¹⁹F-NMR (400 MHz, CDCl₃, CFCl₃) δ (ppm): -64.6 (3F, d, J = 9.2 Hz)

### (Synthesis Example 1: Synthesis of fluorinated diamine represented by following scheme)

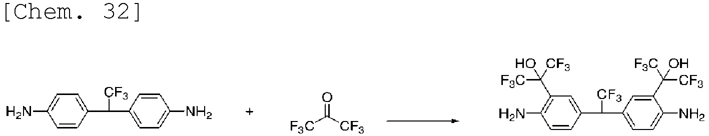

In a 200 mL stainless steel autoclave reactor equipped with a pressure gauge, a thermometer protection tube, a gas inlet tube, and a stirring motor, 20.1 g (75.4 mmol) of 1,1,1-trifluoro-2,2-bis(4-aminophenyl)ethane obtained in Preparation Example 2, 100 g of hexafluoroisopropanol, and 0.11 g (0.75 mmol) of trifluoromethanesulfonic acid were weighed. While cooling the reactor with a dry ice/acetone bath, 26.3 g (158 mmol) of hexafluoroacetone was introduced thereto through the gas inlet tube. Next, the mixture was heated in a 120°C oil bath, and reacted at an absolute pressure of 0.5 to 0.6 MPa for 20 hours. After the reaction, the reaction solution was concentrated with an evaporator to obtain a crude reaction product. Crystals were precipitated by sequentially dropping 40 g of toluene and 31 g of n-heptane to the crude reaction product, thereby obtaining the target product 1,1,1-trifluoro-2,2-bis(3-(1-hydroxy-1-trifluoromethyl-2,2,2-tri fluoroethyl)-4-aminophenyl)ethane in an amount of 29.6 g, yield of 66%, and purity of 97.9% (GC) as a white solid.

### [NMR data]

1,1,1-trifluoro-2,2-bis(3-(1-hydroxy-1-trifluoromethyl-2,2, 2-trifluoroethyl)-4-aminophenyl)ethane:
¹H-NMR (400 MHz, CD₃CN) δ (ppm) : 4.78 (1H, q, J=10.3Hz), 4.85 (2H, br-s), 6.86 (2H, d, J = 6.9 Hz), 7.22 (2H, dd, J = 8.5, 1.1 Hz), 7.32 (2H, d, J = 1.1 Hz)
¹⁹F-NMR (400 MHz, CD₃CN) δ (ppm) : -67.2 (3F, d, J= 8.7 Hz), -75.3 (12F, s)

### (Synthesis Example 2: Synthesis of fluorinated diamine represented by following scheme)

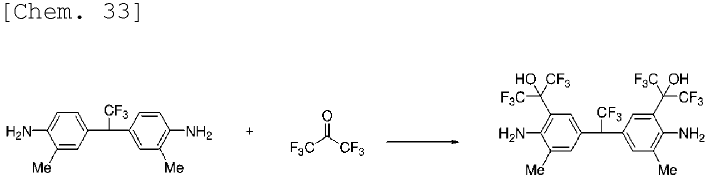

In a 100 mL stainless steel autoclave reactor equipped with a pressure gauge, a thermometer protection tube, a gas inlet tube, and a stirring motor, 9.6 g (32.7 mmol) of
1,1,1-trifluoro-2,2-bis(3-methyl-4-aminophenyl)ethane obtained in Preparation Example 3, 50 g of hexafluoroisopropanol, and 0.25 g (1.6 mmol) of trifluoromethanesulfonic acid were weighed. While cooling the reactor with a dry ice/acetone bath, 12.5 g (75.3 mmol) of hexafluoroacetone was introduced thereto through the gas inlet tube. Next, the mixture was heated in a 105°C oil bath, and reacted at an absolute pressure of 0.3 to 0.4 MPa for 20 hours. The solid obtained by concentrating the reaction solution with an evaporator was recrystallized from 110 g of toluene, thereby obtaining the target product
1,1,1-trifluoro-2,2-bis(3-(1-hydroxy-1-trifluoromethyl-2,2,2-tri fluoroethyl)-5-methyl-4-aminophenyl)ethane in an amount of 17.9 g, yield of 87%, and purity of 98.5% (GC) as a white solid.

### [NMR data]

1,1,1-trifluoro-2,2-bis(3-(1-hydroxy-1-trifluoromethyl-2,2, 2-trifluoroethyl)-5-methyl-4-aminophenyl)ethane:
¹H-NMR (400 MHz, CD₃CN) δ (ppm) : 2.17 (6H, s), 4.45 (2H, br-s), 4.77 (1H, q, J = 10.3 Hz), 7.25 (2H, s), 7.32 (2H, s)
¹⁹F-NMR (400 MHz, CD₃CN) δ (ppm) : -67.0 (3F, d, J= 8.7 Hz), -75.4 (12F, s)

### (Synthesis Example 3: Synthesis of fluorinated diamine represented by following scheme)

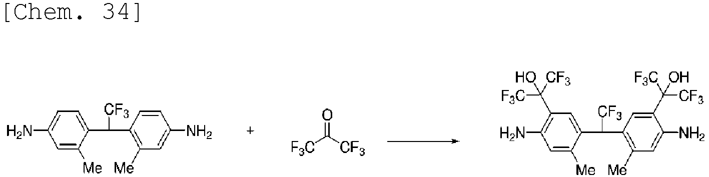

In a 200 mL stainless steel autoclave reactor equipped with a pressure gauge, a thermometer protection tube, a gas inlet tube, and a stirring motor, 20.0 g (67.8 mmol) of
1,1,1-trifluoro-2,2-bis(2-methyl-4-aminophenyl)ethane obtained in Preparation Example 4, 120 g of hexafluoroisopropanol, and 0.51 g (3.1 mmol) of trifluoromethanesulfonic acid were weighed. While cooling the reactor with a dry ice/acetone bath, 25.9 g (156 mmol) of hexafluoroacetone was introduced thereto through the gas inlet tube. Next, the mixture was heated in a 110°C oil bath, and reacted at an absolute pressure of 0.4 MPa for 18 hours. The solid obtained by concentrating the reaction solution with an evaporator was dispersed and washed using 38 g of ethyl acetate and 86 g of n-heptane, thereby obtaining the target product
1,1,1-trifluoro-2,2-bis(3-(1-hydroxy-1-trifluoromethyl-2,2,2-tri fluoroethyl)-6-methyl-4-aminophenyl)ethane in an amount of 34.2 g, yield of 81%, and purity of 98.7% (GC) as a pale violet solid.

### [NMR data]

1,1,1-trifluoro-2,2-bis(3-(1-hydroxy-1-trifluoromethyl-2,2, 2-trifluoroethyl)-6-methyl-4-aminophenyl)ethane:
¹H-NMR (400 MHz, CD₃CN) δ (ppm): 2.15 (6H, s), 4.74 (2H, br-s), 5.00 (1H, q, J = 9.6 Hz), 6.71 (2H, s), 7.26 (2H, s)
¹⁹F-NMR (400 MHz, CD₃CN) δ (ppm) : -66.2 (3F, d, J= 8.7 Hz), -75.5 (6F, s), -75.8 (6F, s)

### (Synthesis Example 4: Synthesis of fluorinated diamine represented by following scheme)

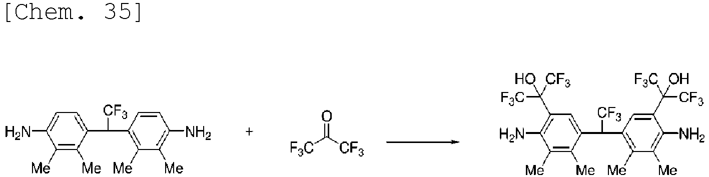

In a 200 mL stainless steel autoclave reactor equipped with a pressure gauge, a thermometer protection tube, a gas inlet tube, and a stirring motor, 20.1 g (62.3 mmol) of
1,1,1-trifluoro-2,2-bis(2,3-dimethyl-4-aminophenyl)ethane obtained in Preparation Example 5, 100 g of hexafluoroisopropanol, and 0.47 g (3.1 mmol) of trifluoromethanesulfonic acid were weighed. While cooling the reactor with a dry ice/acetone bath, 23.8 g (143 mmol) of hexafluoroacetone was introduced thereto through the gas inlet tube. Next, the mixture was heated in a 120°C oil bath, and reacted at an absolute pressure of 0.6 MPa for 20 hours. The solid obtained by concentrating the reaction solution with an evaporator was recrystallized from 32 g of ethyl acetate and 57 g of n-heptane, thereby obtaining the target product
1,1,1-trifluoro-2,2-bis(3-(1-hydroxy-1-trifluoromethyl-2,2,2-tri fluoroethyl)-5,6-dimethyl-4-aminophenyl)ethane in an amount of 31.4 g, yield of 77%, and purity of 99.6% (GC) as a white solid.

### [NMR data]

1,1,1-trifluoro-2,2-bis(3-(1-hydroxy-1-trifluoromethyl-2,2, 2-trifluoroethyl)-5,6-dimethyl-4-aminophenyl)ethane:
¹H-NMR (400 MHz, CD₃CN) δ (ppm) : 2.15 (6H, s), 2.18 (6H, s), 4.51 (2H, br-s), 5.35 (1H, q, J = 9.4 Hz), 7.29 (2H, s)
¹⁹F-NMR (400 MHz, CD₃CN) δ (ppm) : -65.6 (3F, d, J= 8.7 Hz), -75.8 (6F, s), -76.0 (6F, s)

### <Synthesis of polyamide, cyclization (curing) of polyamide, and evaluation>

### (Example 1: Synthesis of polyamide represented by following scheme, cyclization of polyamide, and evaluation)

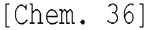

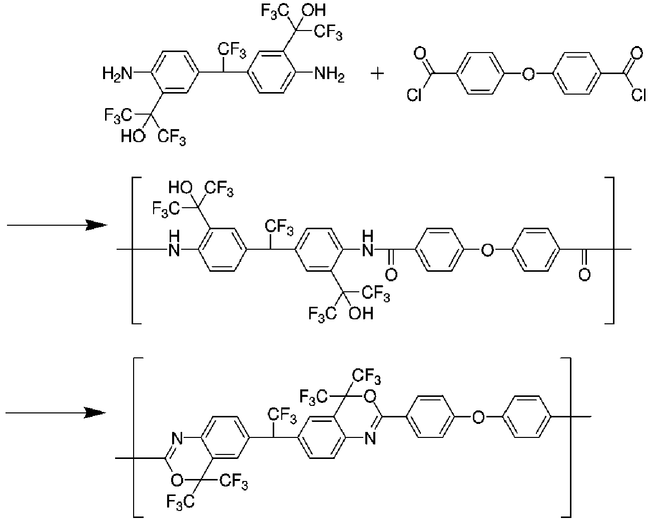

In a 500 mL three-necked flask equipped with a nitrogen inlet tube and a stirrer under ice cooling, 12.0 g (20 mmol) of 1,1,1-trifluoro-2,2-bis(3-(1-hydroxy-1-trifluoromethyl-2,2,2-tri fluoroethyl)-4-aminophenyl)ethane (HFA-BIS-A-EF), which is the fluorinated diamine obtained in Synthesis Example 1, and 5.9 g (20 mmol) of 4, 4-oxybis(benzoylchloride) (hereinafter, may be referred to as OBBC) were added, and 3.6 g (46 mmol) of pyridine as a base and 60 g of N-methylpyrrolidone (NMP) as an organic solvent were added thereto. Under a nitrogen atmosphere, the mixture was stirred at room temperature (25°C) for 5 hours to obtain a reaction solution. Thereafter, the reaction solution was filtered under pressure to prepare a solution of polyamide. As a result of GPC measurement of the solution, Mw was 54500 and Mw/Mn was 2.1.

The prepared solution of polyamide was applied onto a glass substrate using a spin coater. Thereafter, under a nitrogen atmosphere, heating was performed at a temperature of 110°C for 10 minutes as a drying step, and then heating at 200°C for 30 minutes and at 300°C for 2 hours was performed as a curing step to obtain a film (cured film) on the glass substrate. Thereafter, the film was cooled to room temperature, and peeled off from the glass substrate to obtain a film-like cured film (insulation). In a case where a film thickness of the cured film was measured, it was 15 um. In addition, according to an FT-IR measurement result of the cured film, there was an absorption specific to the cyclized polyamide at 1645 cm⁻¹. That is, it was confirmed that the cured film contained cyclized polyamide.

In addition, in a case where a portion of the film after the above-described drying step and before the curing step was taken with a spatula, and the curing temperature was measured, it was 227°C.

In addition, Td₅ of the cured film (containing the cyclized polyamide) was 550°C, the relative permittivity was 2.6, and the dielectric loss tangent was 0.0034.

### (Example 2: Synthesis of polyamide, cyclization of polyamide, and evaluation)

A solution of polyamide was prepared in the same manner as in Example 1, except that the fluorinated diamine (HFA-BIS-3AT-EF) obtained in Synthesis Example 2 was used instead of HFA-BIS-A-EF. As a result of GPC measurement of the solution, Mw was 16000 and Mw/Mn was 1.8.

In addition, the film thickness of the cured film obtained in the same manner as in Example 1 was 33 pm, and Td₅ of the cured film (cyclized polyamide) was 520°C.

### (Example 3: Synthesis of polyamide, cyclization of polyamide, and evaluation)

A solution of polyamide was prepared in the same manner as in Example 1, except that a mixture of the fluorinated diamine (HFA-BIS-3AT-EF) obtained in Synthesis Example 2 and HFA-MDA having the following chemical structure (molar ratio of the mixture was HFA-BIS-3AT-EF:HFA-MDA= 4:1) was used instead of HFA-BIS-A-EF. As a result of GPC measurement of the solution, Mw was 33700 and Mw/Mn was 1.9.

In addition, the film thickness of the cured film obtained in the same manner as in Example 1 was 23 pm, and Td₅ of the cured film (cyclized polyamide) was 520°C.

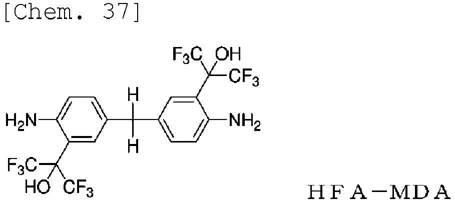

### (Example 4: Synthesis of polyamide, cyclization of polyamide, and evaluation)

A solution of polyamide was prepared in the same manner as in Example 3, except that the fluorinated diamine (HFA-BIS-2AT-EF) obtained in Synthesis Example 3 was used instead of HFA-BIS-3AT-EF (just to be sure, only HFA-BIS-3AT-EF of the two diamines HFA-BIS-3AT-EF and HFA-MDA in Example 3 was replaced with HFA-BIS-2AT-EF). As a result of GPC measurement of the solution, Mw was 68100 and Mw/Mn was 2.2.

In addition, the film thickness of the cured film obtained in the same manner as in Example 1 was 27 pm, and Td₅ of the cured film (cyclized polyamide) was 405°C.

### (Example 5: Synthesis of polyamide, cyclization of polyamide, and evaluation)

A solution of polyamide was prepared in the same manner as in Example 3, except that a mixture of OBBC, IPC, and TPC (molar ratio was OBBC:IPC:TPC = 9: 0.5:0.5; chemical structures of IPC and TPC are as follows) was used instead of OBBC. As a result of GPC measurement of the solution, Mw was 37100 and Mw/Mn was 1.8.

In addition, the film thickness of the cured film obtained in the same manner as in Example 1 was 20 pm, and Td₅ of the cured film (cyclized polyamide) was 510°C.

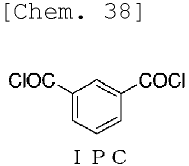

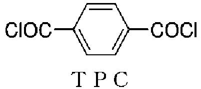

### (Example 6: Synthesis of polyamide, cyclization of polyamide, and evaluation)

A solution of polyamide was prepared in the same manner as in Example 5, except that the fluorinated diamine (HFA-BIS-2AT-EF) obtained in Synthesis Example 3 was used instead of HFA-BIS-3AT-EF. As a result of GPC measurement of the solution, Mw was 73700 and Mw/Mn was 2.0.

In addition, the film thickness of the cured film obtained in the same manner as in Example 1 was 26 pm, and Td₅ of the cured film (cyclized polyamide) was 435°C.

### (Comparative Example 1: Synthesis of polyamide represented by following scheme, cyclization of polyamide, and evaluation)

Polyhydroxyamide was obtained in the same manner as in Example 1, except that
2,2-bis(3-amino-4-hydroxyphenyl)-1,1,1,3,3,3-hexafluoropropane (BIS-AP-AF) was used instead of HFA-BIS-A-EF, and the
polyhydroxyamide was heated to obtain a cured film (containing polybenzoxazole).

A curing temperature of the polyhydroxyamide was 289°C. In addition, the cured film of polybenzoxazole had a dielectric constant of 2.9 and a dielectric loss tangent of 0.0055.

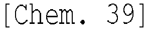

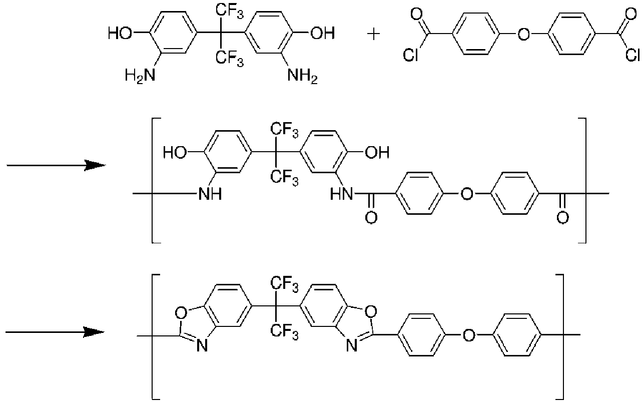

From the comparison between Example 1 and Comparative Example 1, it is understood that a cyclized polyamide having a -CH(CF₃)-structure is obtained under curing conditions of a lower temperature than polybenzoxazole having a -C(CF₃)₂- structure. In addition, it is understood that the cyclized polyamide having a -CH(CF₃)- structure exhibits more excellent low dielectric properties than the polybenzoxazole having a "-C(CF₃)₂-" structure.

Furthermore, heat resistance of the cyclized polyamides having a -CH(CF₃)- structure of Examples 1 to 6 is good.

Priority is claimed on Japanese Patent Application No. 2020-132901, filed August 5, 2020, the disclosure of which is incorporated herein by reference.

## Claims

1. A fluorinated diamine represented by General Formula [1A] or a salt of the fluorinated diamine, 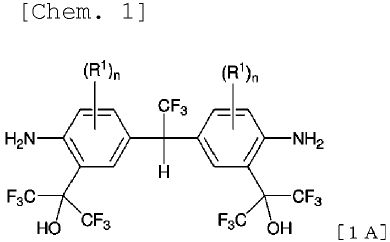 in General Formula [1A],
in a case where a plurality of R¹'s are present, the plurality of R¹'s are each independently at least one selected from the group consisting of an alkyl group, an alkoxy group, a halogen atom, a haloalkyl group, and a haloalkoxy group, and
two n's are each independently an integer of 0 to 3.

2. The fluorinated diamine or the salt of the fluorinated diamine according to claim 1,
wherein the fluorinated diamine or the salt of the fluorinated diamine is represented by General Formula [1A-1], 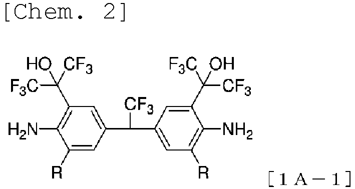
in General Formula [1A-1], two R's are each independently an alkyl group having 1 to 6 carbon atoms.

3. The fluorinated diamine or the salt of the fluorinated diamine according to claim 1,
wherein the fluorinated diamine or the salt of the fluorinated diamine is represented by General Formula [1A-2], 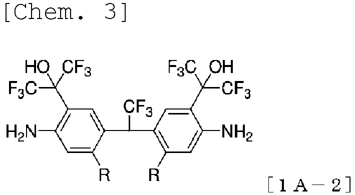
in General Formula [1A-2], two R's are each independently an alkyl group having 1 to 6 carbon atoms.

4. The fluorinated diamine or the salt of the fluorinated diamine according to claim 1,
wherein the fluorinated diamine or the salt of the fluorinated diamine is represented by General Formula [1A-3], 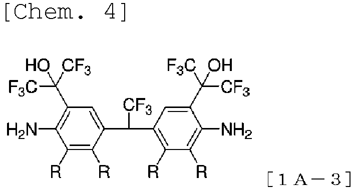
in General Formula [1A-3], four R's are each independently an alkyl group having 1 to 6 carbon atoms.

5. The fluorinated diamine or the salt of the fluorinated diamine according to claim 1,
wherein the fluorinated diamine or the salt of the fluorinated diamine is represented by Formula [1A-4],

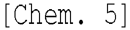

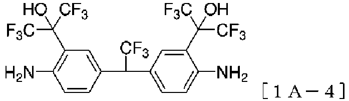

6. The fluorinated diamine or the salt of the fluorinated diamine according to claim 1 or 2,
wherein the fluorinated diamine or the salt of the fluorinated diamine is represented by Formula [1A-5], 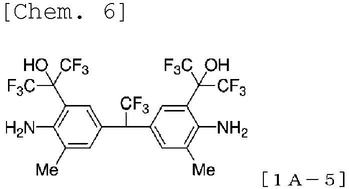
in Formula [1A-5], Me represents a methyl group.

7. The fluorinated diamine or the salt of the fluorinated diamine according to claim 1 or 3,
wherein the fluorinated diamine or the salt of the fluorinated diamine is represented by Formula [1A-6], 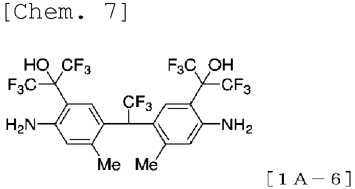
in Formula [1A-6], Me represents a methyl group.

8. The fluorinated diamine or the salt of the fluorinated diamine according to claim 1 or 4,
wherein the fluorinated diamine or the salt of the fluorinated diamine is represented by Formula [1A-7], 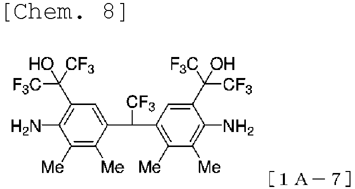
in Formula [1A-7], Me represents a methyl group.

9. A method for producing the fluorinated diamine or the salt of the fluorinated diamine according to any one of claims 1 to 8, the method comprising:
a step of adding hexafluoroacetone or an equivalent of hexafluoroacetone to an aromatic diamine compound represented by General Formula [2], 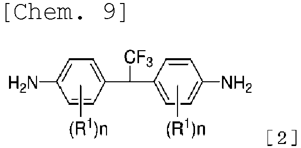
in General Formula [2], definitions of R¹ and n are the same as definitions of R¹ and n in General Formula [1A].

10. A polyamide comprising:
a structural unit represented by General Formula [1B], 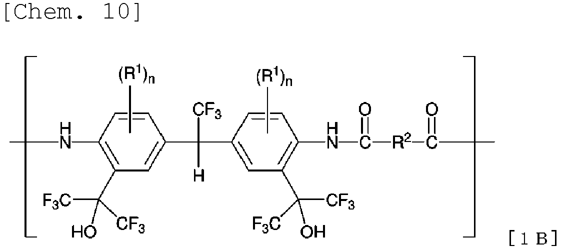
in General Formula [1B],
in a case where a plurality of R¹'s are present, the plurality of R¹'s are each independently at least one selected from the group consisting of an alkyl group, an alkoxy group, a halogen atom, a haloalkyl group, and a haloalkoxy group,
R² is a divalent organic group, and
two n's are each independently an integer of 0 to 3.

11. The polyamide according to claim 10,
wherein R² is a divalent organic group containing an aromatic ring.

12. The polyamide according to claim 10 or 11,
wherein R² is at least one divalent organic group selected from the following groups,

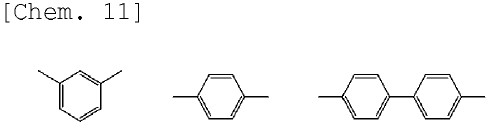

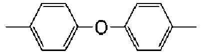

13. The polyamide according to any one of claims 10 to 12,
wherein a weight-average molecular weight is 1,000 or more and 1,000,000 or less.

14. A polyamide solution comprising:
the polyamide according to any one of claims 10 to 13; and
an organic solvent.

15. The polyamide solution according to claim 14,
wherein the organic solvent includes at least one selected from the group consisting of an amide-based solvent, an ether-based solvent, an aromatic solvent, a halogen-based solvent, and a lactone-based solvent.

16. The polyamide solution according to claim 14 or 15,
wherein a concentration of the polyamide is 0.1% by mass or more and 50% by mass or less.

17. A cyclized polyamide comprising:
a structural unit represented by General Formula [1C], 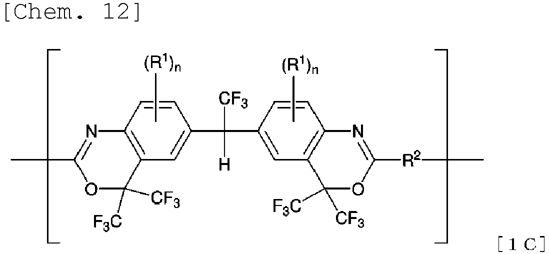
in General Formula [1C],
in a case where a plurality of R¹'s are present, the plurality of R¹'s are each independently at least one selected from the group consisting of an alkyl group, an alkoxy group, a halogen atom, a haloalkyl group, and a haloalkoxy group,
R² is a divalent organic group, and
two n's are each independently an integer of 0 to 3.

18. The cyclized polyamide according to claim 17,
wherein R² is a divalent organic group containing an aromatic ring.

19. The cyclized polyamide according to claim 17 or 18,
wherein R² is at least one divalent organic group selected from the following groups,

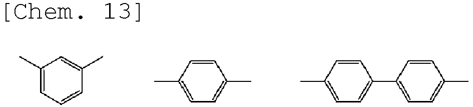

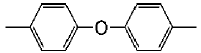

20. An insulation for a high-frequency electronic component, comprising:
the cyclized polyamide according to any one of claims 17 to 19.

21. The insulation for a high-frequency electronic component according to claim 20,
wherein a 5%-weight-loss temperature Td₅ is 350° or higher.

22. The insulation for a high-frequency electronic component according to claim 20 or 21,
wherein a dielectric loss tangent at a frequency of 28 GHz is 0.012 or less.

23. The insulation for a high-frequency electronic component according to any one of claims 20 to 22,
wherein a relative permittivity at a frequency of 28 GHz is 3.1 or less.

24. A high-frequency electronic component comprising:
the insulation for a high-frequency electronic component according to any one of claims 20 to 23.

25. A high-frequency appliance comprising:
the high-frequency electronic component according to claim 24.

26. An insulating material for producing a high-frequency electronic component, comprising:
the polyamide according to any one of claims 10 to 13.

27. The insulating material for producing a high-frequency electronic component according to claim 26,
wherein a weight-average molecular weight of the polyamide is 500,000 or less.

28. A production method for producing the polyamide according to any one of claims 10 to 13, the production method comprising:
a step of polycondensing a fluorinated diamine represented by General Formula [1A] or a salt of the fluorinated diamine with a dicarboxylic acid represented by General Formula [DC1] or [DC2] or a derivative of the dicarboxylic acid, 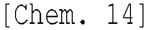 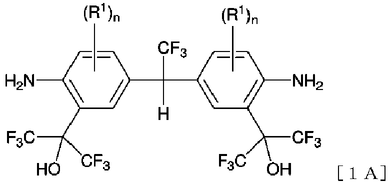
in General Formula [1A],
in a case where a plurality of R¹'s are present, the plurality of R¹'s are each independently at least one selected from the group consisting of an alkyl group, an alkoxy group, a halogen atom, a haloalkyl group, and a haloalkoxy group, and
two n's are each independently an integer of 0 to 3, 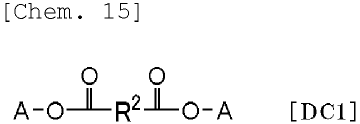
in General Formula [DC1], R² is the same as R² in General Formula [1B], and two A's each independently represent a hydrogen atom, an alkyl group having 1 to 10 carbon atoms, or an aromatic hydrocarbon group having 6 to 10 carbon atoms, 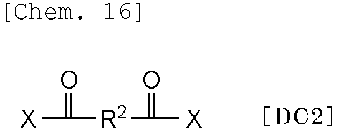
in General Formula [DC2], R² is the same as R² in General Formula [1B], and two X's each independently represent a fluorine atom, a chlorine atom, a bromine atom, an iodine atom, or an active ester group.

29. A production method for producing the cyclized polyamide according to any one of claims 17 to 19, the production method comprising:
a first step of producing a polyamide by the production method according to claim 28; and
a second step of dehydrating and ring-closing the polyamide obtained in the first step.

30. A method for producing an insulation for a high-frequency electronic component, the method comprising:
a coating step of applying the polyamide solution according to any one of claims 14 to 16 onto a supporting base material;
a drying step of drying the solvent contained in the applied polyamide solution to obtain a resin film including the polyamide; and
a heating step of heat-treating the resin film to form a cured film.

31. The method for producing an insulation for a high-frequency electronic component according to claim 30,
wherein the supporting base material is at least one selected from the group consisting of glass, a silicon wafer, stainless steel, alumina, copper, nickel, polyethylene terephthalate, polyethylene glycol terephthalate, polyethylene glycol naphthalate, polycarbonate, polyimide, polyamide-imide, polyetherimide, polyetheretherketone, polypropylene, polyether sulfone, polyethylene terephthalate, polyphenylene sulfone, and polyphenylene sulfide.

32. The method for producing an insulation for a high-frequency electronic component according to claim 30 or 31,
wherein a film thickness of the cured film is 1 pm or more and 1000 um or less.

33. The method for producing an insulation for a high-frequency electronic component according to any one of claims 30 to 32,
wherein the drying step is performed at a temperature of 50°C or higher and 250°C or lower.

34. The method for producing an insulation for a high-frequency electronic component according to any one of claims 30 to 33,
wherein the heating step is performed at a temperature of 100°C or higher and 400°C or lower.
